# FASCICULE DE BREVET EUROPEEN

(11) **EP 0 850 235 B1**
(45) Date de publication et mention de la délivrance du brevet: **01.12.1999**
(21) Numéro de dépôt: 96931112.5
(22) Date de dépôt: 12.09.1996
(51) Int. Cl.: C07D 401/06, A61K 31/47, C07D 495/04

(54) **DERIVES DE QUINOLEIN-2(1H)-ONE COMME ANTAGONISTES DE LA SEROTONINE**
CHINOLIN-2-ON DERIVATE ALS SEROTONIN ANTAGONISTEN
QUINOLEIN-2(1H)-ONE DERIVATIVES AS SEROTONIN ANTAGONISTS

(30) Priorité: 15.09.1995 FR 9510815; 21.09.1995 FR 9511083
(43) Date de publication de la demande: 01.07.1998
(73) Titulaire: Sanofi-Synthélabo, 75013 Paris (FR)
(72) Inventeur: Mc CORT, Gary, F-75007 Paris (FR); HOORNAERT, Christian, F-92160 Antony (FR); DELLAC, Geneviève, F-91420 Morangis (FR); ALETRU, Michel, F-75020 Paris (FR)
(74) Mandataire: Thouret-Lemaitre, Elisabeth
(86) Numéro de dépôt international: FR9601401
(87) Numéro de publication internationale: WO9710238

(56) Documents cités:
- EP-A- 0 288 563
- EP-A- 0 389 352
- GB-A- 2 174 703
- JOURNAL OF MEDICINAL CHEMISTRY, vol. 35, no. 26, 1992, WASHINGTON US, pages 4903-4910, XP002002803 JEFF L. HERNDON ET AL.: "Ketanserin analogues:..."
- JOURNAL OF MEDICINAL CHEMISTRY, vol. 32, no. 6, 1989, WASHINGTON US, pages 1147-1156, XP002002750 J.S. NEW ET AL: "The thieno(3,2-c)pyridine and ..."

## Description

La présente invention a pour objet des dérivés de quinoléin-2(1*H*)-one, leur préparation et leur application en thérapeutique.

Les composés de l'invention répondent à la formule (I) 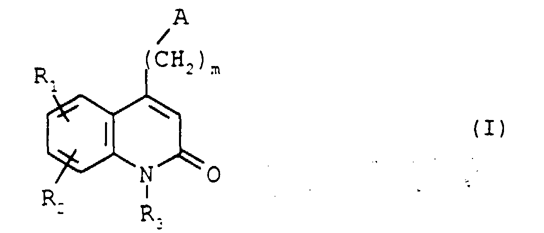 dans laquelle
A représente soit un groupe 4-(thiéno[3,2-*c*]pyridin-4-yl) pipérazin-1-yle, soit un groupe 4-(4-fluorobenzoyl)pipéridin-1-yle,
R₁ et R₂ représentent chacun indépendamment l'un de l'autre, soit un atome d'hydrogène, soit un atome d'halogène, soit un groupe amino, soit un groupe hydroxy, soit un groupe nitro, soit un groupe cyano, soit un groupe (C₁-C₆)alkyle, soit un groupe (C₁-C₆)alcoxy, soit un groupe trifluorométhyle, soit un groupe trifluorométhoxy, soit un groupe -COOH, soit un groupe -COOR₄, soit un groupe -CONH₂, soit un groupe -CONHR₄, soit un groupe -CONR₄R_{5,} soit un groupe -SR₄, soit un groupe -SO₂R₄, soit un groupe -NHCOR₄, soit un groupe -NHSO₂R₄, soit un groupe -N(R₄)₂ où R₄ et R₅ sont chacun un groupe (C₁-C₄)alkyle,
R₃ représente soit un atome d'hydrogène, soit un groupe (C₁-C₄)alkyle, soit un groupe -(CH₂)ₚOH, soit un groupe -(CH₂)ₚNH₂, soit un groupe -(CH₂)ₙCOOH, soit un groupe -(CH₂)ₙCOOR₄, soit un groupe -(CH₂)ₙCONH₂, soit un groupe -(CH₂)ₙCONHOH, soit un groupe -(CH₂)ₚSH, soit un groupe -(CH₂)ₙSO₃H, soit un groupe -(CH₂)ₙSO₂NH₂, soit un groupe -(CH₂)ₙSO₂NHR₄, soit un groupe -(CH₂)ₙSO₂NR₄R₅, soit un groupe -(CH₂)ₙCONHR₄, soit un groupe -(CH₂)ₙCONR₄R₅, soit un groupe -(CH₂)ₚNHSO₂R₄, soit un groupe -(CH₂)ₚNHCOR₄, soit un groupe -(CH₂)ₚOCOR₄ où R₄ et R₅ sont chacun un groupe (C₁-C₄)alkyle, n est égal à 1, 2, 3 ou 4, p est égal à 2, 3 ou 4 et m est égal à 2, 3 ou 4,
ainsi que leurs sels d'addition aux acides ou aux bases pharmaceutiquement acceptables.

Selon l'invention, les composés de formule (I) peuvent être synthétisés selon le schéma 1.
On fait réagir de la 4-(acétyloxy)-2*H*,3*H*-pyrane-2,6-dione avec un composé de formule (II) (dans laquelle R₁ et R₂ sont tels que définis précédemment et R₃ est un atome d'hydrogène ou un groupe (C₁-C₄)alkyle) à température ambiante dans un solvant polaire tel que l'acide acétique. Après séchage, on cyclise le composé de formule (III) ainsi obtenu, en présence d'un acide minéral ou organique, de préférence anhydre, tel que l'acide sulfurique concentré, l'acide polyphosphorique, ou l'acide trifluorométhanesulfonique à une température comprise entre 10 et 150 °C et on obtient un acide 2-oxo-1,2-dihydro-quinoléine-4-acétique, substitué ou non, de formule (IV) que l'on estérifie avec un alcool de formule R₆OH (où R₆ est un groupe (C₁-C₄)alkyle), par n'importe quelle méthode d'estérification, de préférence par l'action du chlorure de thionyle. Ensuite on réduit l'ester de formule (V) ainsi obtenu, par un hydrure dans un solvant aprotique comme, par exemple, l'hydrure de lithium et d'aluminium dans le dioxane ou le borohydrure de sodium en excès dans le tétrahydrofurane au reflux, ou le borohydrure de lithium dans le tétrahydrofurane à température ambiante pour obtenir un alcool de formule (VI) (dans laquelle m est égal à 2) ; on obtient les composés de formule (VI), dans laquelle m est égal à 3 ou 4, à partir de ceux où m est égal à 2 par des techniques d'homologation connues de l'homme du métier. Ensuite on active les composés de formule (VI) (dans laquelle m est égal à 2, 3 ou 4) en composés de formule (VII) (dans laquelle X représente un groupe partant tel un atome de chlore ou de brome) par exemple par réaction avec le chlorure de thionyle dans le chloroforme au reflux ou le dibromotriphénylphosphorane à température ambiante dans le dichloro 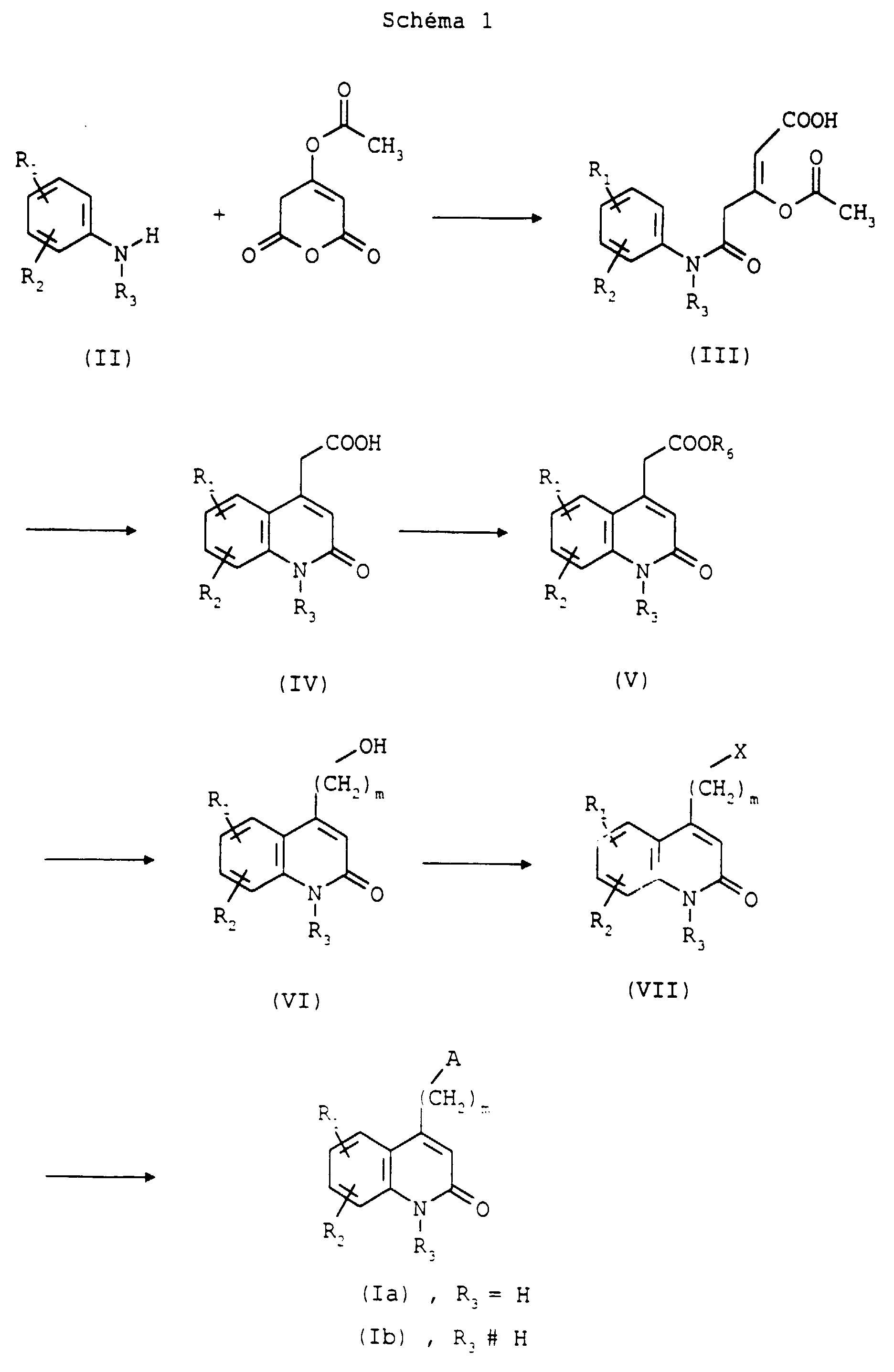 méthane ou en composés de formule (VII) (dans laquelle X représente un groupe partant tel que les groupes méthanesulfonyloxy, trifluorométhanesulfonyloxy ou paratoluènesulfonyloxy), par exemple par réaction avec un anhydride sulfonique ou un chlorure d'acide sulfonique en présence d'une base telle que la pyridine ou la triéthylamine. Finalement on fait réagir les composés de formule (VII) avec la 4-(pipérazin-1-yl)thiéno[3,2-*c*]pyridine ou avec la 4-(4-fluorobenzoyl)pipéridine avec ou sans solvant aprotique ou protique, en présence d'une base inorganique entre 20 °C et 150 °C, de préférence dans l'acétonitrile ou le diméthylformamide en contact avec du bicarbonate de sodium et on obtient un composé de formule (I).

Pour préparer un composé de formule (Ib) (dans laquelle R₃ est différent d'un atome d'hydrogène), on peut réaliser l'alkylation du composé de formule (Ia) correspondant (dans laquelle R₃ représente un atome d'hydrogène) à l'aide d'un agent électrophile du type R₃Br ou R₃I, tel que par exemple le bromoacétate de tert-butyle, la bromométhanesulfonamide, la *N*-méthylbromométhanesulfonamide, la bromoacétamide, la *N*-méthylbromoacétamide, la *N*,*N*-diméthylbromoacétamide ou l'acétate de 2-bromoéthyle en présence d'une base telle que l'hydrure de sodium ou de potassium, dans un solvant aprotique tel que le tétrahydrofurane ou le diméthylformamide, en présence ou non d'un catalyseur de transfert de phase, tel que le bromure de tétrabutylammonium. Ensuite si on veut préparer les composés de formule (Ib) dans laquelle R₃ représente un groupe -(CH₂)ₙCOOH, on réalise une désestérification des composés de formule (Ib) correspondants dans laquelle R₃ représente un groupe -(CH₂)ₙCOOR₄. Si on veut préparer les composés de formule (Ib) dans laquelle R₃ représente un groupe -(CH₂)ₚOH, on réalise une déacétylation des composés de formule (Ib) correspondants dans laquelle R₃ représente un groupe -(CH₂)ₚOCOR₄.

Pour obtenir un composé de formule (I) dans laquelle R₁ et/ou R₂ représentent un groupement cyano, -CONH₂, -COOH, -COOR₄, -SR₄ ou -SO₂R₄ où R₄ est un groupe (C₁-C₄)alkyle, la cyclisation du composé de formule (III) en quinoléinone de formule (IV) étant défavorisée, on conduit plutôt la synthèse des composés de formules (V) et (VI) correspondants selon les schémas 2 et 3.
Selon le schéma 2, on fait réagir un composé de formule (Va) correspondant à un composé de formule (V) (dans laquelle R₁ représente un atome d'iode, R₂ et R₆ sont tels que définis précédemment et R₃ est un atome d'hydrogène ou un groupe (C₁-C₄)alkyle) avec un sel de cyanure en présence d'un sel de cuivre dans un solvant polaire tel que le diméthylformamide ou la *N*-méthylpyrrolidone, ou avec le cyanure de triméthylsilyle en présence d'un catalyseur de palladium, de préfé rence du tétrakis(triphénylphosphine)palladium[0] dans la triéthylamine au reflux pour obtenir un composé de formule (Vb) que l'on peut soit transformer en composé de formule (VId) puis en composé de formule (VIe) (dans laquelle R₇ est un atome d'hydrogène ou un groupe (C₁-C₄)alkyle), soit transformer en dérivé carboxamide de formule (Vc) par des méthodes classiques connues de l'hommme du métier. 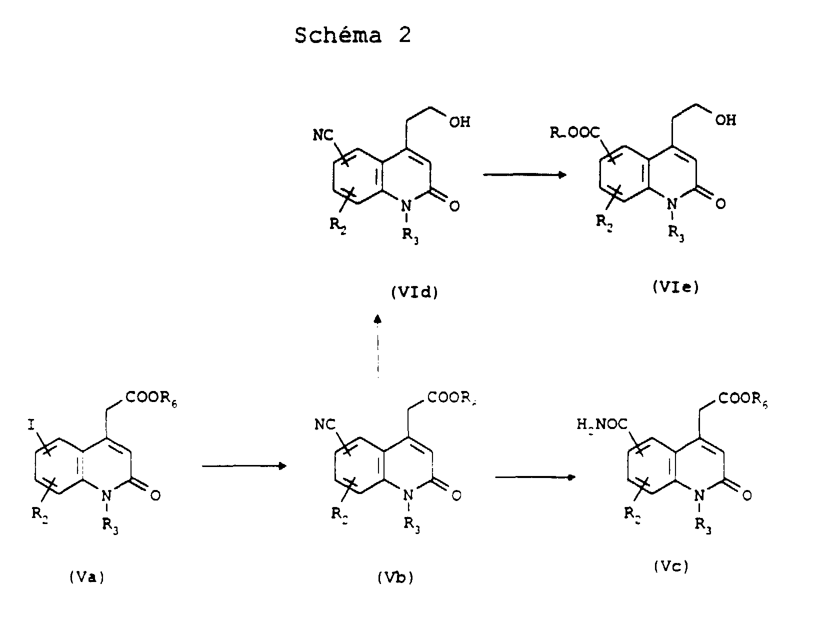 Selon le schéma 3, on fait réagir un composé de formule (VIa) correspondant à un composé de formule (VI) (dans laquelle R₁ représente un atome d'iode, R₂ est tel que défini précédemment, R₃ est un atome d'hydrogène ou un groupe (C₁-C₄)alkyle et m est égal à 2) avec un thiolate tel que le thiométhoxyde de sodium en présence de tétrakis(triphényl phosphine)palladium[0] dans un alcool, tel que l'éthanol, le propanol ou le *n*-butanol pour préparer un composé de formule (VIb) (dans laquelle R₄ est un groupe (C₁-C₄)alkyle) que l'on peut transformer par oxydation en composé de formule (VIc).

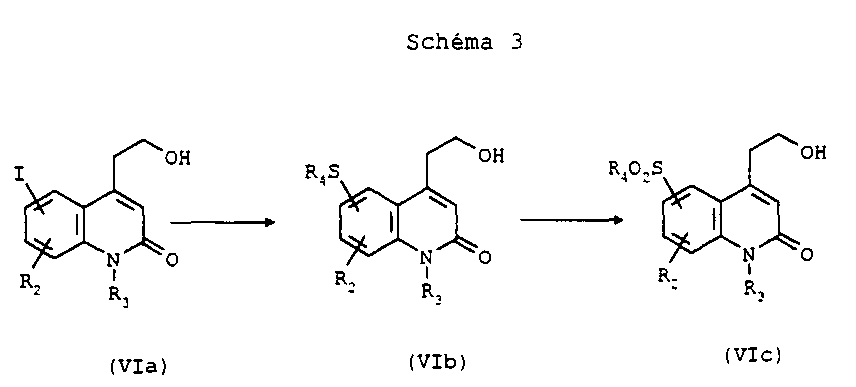

Pour obtenir les composés de formule (I) dans laquelle R₁ et/ou R₂ représentent un groupe nitro, amino, -NHCOR₄, -NHSO₂R₄ ou -N(R₄)₂, R₄ étant un groupe (C₁-C₄)alkyle, on conduit la synthèse des composés de formule (VII) correspondants selon le schéma 4.
On réalise la nitration d'un composé de formule (VIIa) correspondant à un composé de formule (VII) (dans laquelle R₁ est un atome d'hydrogène, X un atome d'halogène et R₃ un atome d'hydrogène ou un groupe (C₁-C₄)alkyle) pour obtenir un composé de formule (VIIb) que l'on transforme en composé de formule (VIIc) par réduction à l'hydrogène, composé que l'on transforme soit en composé de formule (VIId) par réaction avec un chlorure d'acide carboxylique de formule R₄COCl, soit en composé de formule (VIIe) par réaction avec un chlorure d'acide sulfonique de formule R₄SO₂Cl, soit en composé de formule (VIIf) par réaction de *N*-dialkylation. Ensuite on 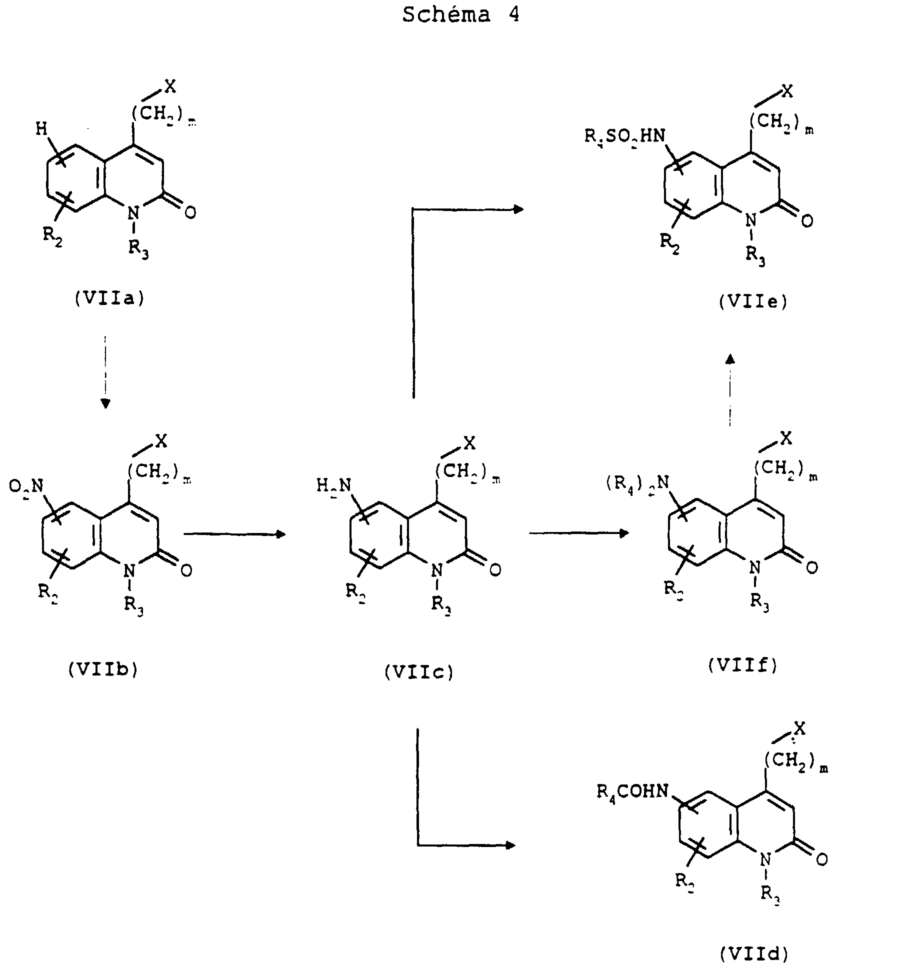 fait réagir ces composés avec la 4-(pipérazin-1-yl)thiéno [3,2-*c*]pyridine ou avec la 4-(4-fluorobenzoyl)pipéridine selon le schéma 1.

Pour préparer les composés de formule (I) dans laquelle R₁ et/ou R₂ représentent un groupe hydroxy, on peut réaliser une désalkylation du composé alcoxylé correspondant de formule (I) (dans laquelle R₁ et/ou R₂ représentent un groupe alcoxyle) dans des conditions classiques connues de l'homme du métier, comme par exemple un traitement à l'acide bromhydrique à 48 %.

Les composés de départ sont disponibles dans le commerce ou décrits dans la littérature ou peuvent être préparés selon des méthodes qui y sont décrites ou qui sont connues de l'homme du métier.

Ainsi la 4-(acétyloxy)-2*H*,3*H*-pyrane-2,6-dione est préparée à partir de l'acide 3-oxo-glutarique selon E.G. FRANDSEN et N. JACOBSEN, *J. Chem. Soc. Perkin* I, pp 933-6 (1978). Le procédé de cyclisation est adapté de ceux décrits dans les demandes de brevets européens EP0364327 et EP0577325. L'introduction d'un nitrile sur les composés de formule (V) est réalisée selon la méthodologie décrite par N. CHANTANI et T. HANAFUSA, *J. Org. Chem.* 51, pp 4714-4716 (1986). La substitution nucléophile aromatique des aryles iodés, par des thiolates est basée sur la méthode de T. MIGITAL et coll. *Bull. Chem. Soc. Japan,* 53, pp 1385 (1980).
La 4-(pipérazin-1-yl)thiéno[3,2-*c*]pyridine est synthétisée selon J.S. New, et coll., *J. Med. Chem.* 32, N°6, pp 1147-56 (1989).

Les exemples qui suivent illustrent l'invention sans la limiter. Les micro-analyses et les spectres IR, RMN et de masse confirment la structure des composés obtenus. Les numéros des composés exemplifiés renvoient à ceux du tableau donné plus loin qui illustre les structures chimiques et les propriétés physiques de quelques composés selon l'invention.
Les rapports (x:y) correspondent au rapport (acide:base).

### Exemple 1 (composé n° 27)

### chlorhydrate de 6-méthoxy-4-[2-[4-(thiéno-[3,2-c]pyridin-4-yl)pipérazin-1-yl]éthyl]quinoléin-2(1H)one (2:1)

1.1. acide 3-(acétyloxy)-5-[(4-méthoxyphényl)méthylamino]-5-oxopent-2-énoïque
   A une solution de 20,0 g (146 mmoles) de N-méthyl-4-méthoxy aniline dans 100 ml d'acide acétique, on ajoute sous agitation forte à température ambiante, 27 g (158 mmoles) de 4-(acétyloxy)-2*H*,3*H*-pyrane-2,6-dione. Après 5 heures d'agitation à température ambiante, on ajoute 700 ml d'eau glacée et on laisse agiter 30 minutes. On obtient un solide beige qui est essoré, lavé à l'eau, trituré dans de l'éther diéthylique et séché sur pentoxyde de phosphore à 40 °C pendant 24 heures.
   On obtient 28,1 g de produit sous forme d'un solide.
   Point de fusion = 85-88 °C.
   Rendement = 76 %
1.2. acide 6-méthoxy-2-oxo-1,2-dihydroquinoléine-4-acétique A 70 ml d'acide sulfurique (96-97 %) à température ambiante, on ajoute par petites portions 41 g (133 mmoles) d'acide 3-(acétoxy)-5-[(4-méthoxyphényl)méthylamino]-5-oxopent-2-énoïque, puis on chauffe sous agitation à 80 °C pendant 1 heure 30. Après refroidissement, on verse le milieu réactionnel dans 100 g de glace et 100 ml d'eau, on agite 15 minutes et on essore le solide que l'on lave abondamment à l'eau avant de le sécher 48 heures à 50 °C. On recueille 14,9 g d'un mélange d'acide 6-méthoxy-1-méthyl-2-oxo-1,2-dihydroquinoléine-4-acétique et d'acide 6-méthoxy-2-oxo-1,2-dihydroquinoléine-4-acétique.
   Rendement = 45 %.
1.3. 6-méthoxy-2-oxo-1,2-dihydroquinoléine-4-acétate de méthyle
   On ajoute goutte à goutte, 16 ml (219 mmoles) de chlorure de thionyle à une suspension agitée de 16,8 g (68 mmoles) d'un mélange d'acide 6-méthoxy-1-méthyl-2-oxo-1,2-dihydroquinoléine-4-acétique et d'acide 6-méthoxy-2-oxo-1,2-dihydroquinoléine-4 acétique dans 250 ml de méthanol à température ambiante, puis on maintient l'agitation pendant 16 heures. On évapore le solvant sous vide et on reprend le résidu dans 400 ml de dichlorométhane. On lave avec une solution saturée en hydrogénocarbonate de sodium, puis avec de l'eau et on sèche la phase organique sur sulfate de sodium. Après filtration et concentration, on obtient 12,6 g d'un mélange des deux esters (71 %). On sépare les deux esters par chromatoflash sur silice en éluant par un mélange méthanol/dichlorométhane (3:97).
   On obtient 4,0 g de 6-méthoxy-1-méthyl-2-oxo-1,2-dihydroquinoléine-4 acétate de méthyle,
   Point de fusion = 129-130 °C
   et 7,8 g de 6-méthoxy-2-oxo-1,2-dihydroquinoléine-4-acétate de méthyle
   Point de fusion = 223-224 °C
1.4. 4-(2-hydroxyéthyl)-6-méthoxyquinoléin-2(1*H*)-one A une suspension de 3,1 g (12,5 mmoles) de 6-méthoxy-2-oxo-1,2-dihydroquinoléine acétate de méthyle dans 100 ml de tétrahydrofurane sec et 1 ml de méthanol, à température ambiante, on ajoute 1,4 g de borohydrure de sodium (37 mmoles) et on chauffe le milieu réactionnel au reflux pendant 16 heures. Après refroidissement à 5 °C, on ajoute 1 ml de méthanol goutte à goutte, puis après 30 minutes, 0,5 g de borohydrure de sodium et on chauffe le milieu réactionnel encore pendant 8 heures. Après refroidissement et traitement avec 5 ml de méthanol, on évapore les solvants et on reprend le résidu par 200 ml de dichlorométhane et 100 ml d'acide chlorhydrique 1 N. On sépare la phase organique, on la lave à l'eau et on la sèche sur sulfate de sodium. Après filtration et concentration sous vide, on obtient 1,95 g de l'alcool attendu.
   Rendement = 72 %
1.5. 4-12-chloroéthyl)-6-méthoxyquinoléin-2(1*H*)-one A une suspension de 3,11 g (14,2 mmoles) de 4-(2-hydroxyéthyl)-6-méthoxyquinoléine-2-(1*H*)-one dans 50 ml de chloroforme et 3 gouttes de diméthylformamide, on additionne en agitant 3,4 ml (46,6 mmoles) de chlorure de thionyle, à température ambiante. On chauffe la suspension au reflux pendant 14 heures (solubilisation totale). Après refroidissement à température ambiante, on ajoute, goutte à goutte, 50 ml d'eau au milieu réactionnel et on laisse agiter 30 minutes. On récupère la phase organique, on la décante, on la lave à l'eau, on la sèche sur sulfate de magnésium et on filtre. On concentre le filtrat sous vide.
   On obtient 3,2 g d'un solide jaune pâle.
   Point de fusion = 231-232 °C
   Rendement = 94 %
1.6. chlorhydrate de 6-méthoxy-4-[2-[4-(thiéno-[3,2-*c*] pyridin-4-yl)pipérazin-1-yl]éthyl]quinoléin-2 (1*H*)-one (2:1)
   On ajoute 1,2 g (5 mmoles) de (2-chloroéthyl)-6-méthoxy quinoléin-2(1*H*)one à une suspension de 1,2 g (5,5 mmoles) de 4-(pipérazin-1-yl)thiéno[3,2-*c*]pyridine et 0,44 g (5,25 mmoles) d'hydrogénocarbonate de sodium dans 15 ml d'acétonitrile, puis on chauffe le mélange réactionnel au reflux pendant 10 heures. Après évaporation sous vide du solvant, on reprend le résidu dans 100 ml de dichlorométhane et on lave successivement avec une solution aqueuse saturée en bicarbonate de sodium puis avec de l'eau. Après séchage sur sulfate de sodium, filtration et condensation du filtrat, on purifie le produit brut par chromatoflash sur silice en éluant par un mélange méthanol/dichlorométhane (5:95) contenant des traces d'ammoniaque.
   On obtient 0,50 g du produit sous forme de base.
   Rendement = 24 %
   On prépare le dichlorhydrate dans un mélange méthanol/acide chlorhydrique/éther.
   Point de fusion = 254 °C (décomposition)

### Exemple 2 (composé n° 28)

### chlorhydrate de 4-[2-[4-(4-fluorobenzoyl)pipéridin-1-yl] éthyl]-6-méthoxyquinoléin-2(1H)-one (1:1)

On chauffe au reflux pendant 8,5 heures un mélange de 1,1 g (4,6 mmoles) de 4-(2-chloroéthyl)-6-méthoxyquinoléin-2(1*H*)-one, 1,0 g (5,5 mmoles) de 4-(4-fluorobenzoyl)pipéridine et 0,38 g (4,6 mmoles) d'hydrogénocarbonate de sodium dans 20 ml d'acétonitrile. On évapore ensuite le milieu réactionnel à sec et on purifie le produit brut par chromatoflash sur silice en éluant par un mélange méthanol/dichlorométhane (5:95) contenant des traces d'ammoniaque.
On obtient 0,53 g du produit attendu sous forme de base.
Rendement = 30 %
On prépare le chlorhydrate dans un mélange méthanol/acide chlorhydrique.
Point de fusion = 237 °C (décomposition)

### Exemple 3 (composé n° 4)

### chlorhydrate de 6-chloro-4-[2-[4-(4-fluorobenzoyl)pipéridin-1-yl]éthyl]-1-méthylquinoléin-2(1H)-one (1:1)

3.1. acide 3-(acétyloxy)-5-[(4-chlorophényl)méthylamino]-5-oxo-pent-2-énoïque
   A une solution agitée de 15,0 g (106 mmoles) de 4-chloro-N-méthylbenzèneamine dans 40 ml d'acide acétique pur, on ajoute par petites portions, 19,8 g (116 mmoles) de 4-(acétyloxy)2*H*,3*H*-pyrane-2,6-dione. On agite le milieu réactionnel pendant 3 heures à 35 °C. On laisse refroidir à température ambiante et on dilue dans 10 ml d'eau glacée. On essore le solide, on le lave abondamment à l'eau et on le sèche à 40 °C pendant 48 heures.
   On obtient 25,5 g du composé attendu sous forme d'un solide amorphe que l'on utilise tel quel dans l'étape suivante.
   Rendement = 77 %
3.2. acide 6-chloro-1-méthyl-2-oxo-1,2-dihydroquinoléine-4-acétique
   On verse, par petites portions 25,5 g (81,8 mmoles) d'acide 3-(acétyloxy)-5-[(4-chlorophényl)méthylamino]-5-oxopent-2-énoïque dans 40 ml d'acide sulfurique concentré à température ambiante sous forte agitation, puis on chauffe le milieu réactionnel à 85 °C pendant 60 minutes. Après refroidissement, on verse cette solution dans un mélange de 500 g de glace et 500 ml d'eau. On essore le solide gris ainsi obtenu, on le lave à l'eau puis on le triture dans l'éther et on le sèche pendant 24 heures à 40 °C.
   On obtient 9,47 g du produit attendu que l'on utilise tel quel dans l'étape suivante.
   Rendement = 46 %
3.3. 6-chloro-1-méthyl-1,2-dihydroquinoléine-4-acétate de méthyle
   On ajoute goutte à goutte, en 30 minutes environ, 11 ml (147 mmoles) de chlorure de thionyle à une suspension agitée de 12,5 g (49 mmoles) d'acide 6-chloro-1-méthyl-2-oxo-1,2-dihydroquinoléine-4-acétique dans 150 ml de méthanol. On agite pendant 17 heures à la température ambiante et on chasse le solvant sous vide. On dissout le résidu dans 400 ml de dichlorométhane, puis on le lave avec une solution aqueuse saturée en bicarbonate de sodium puis à l'eau. Après séchage sur sulfate de sodium, on filtre et on condense le filtrat. On obtient 11,16 g de produit attendu.
   Rendement = 85 %
   Point de fusion = 99-101 °C
3.4. 6-chloro-4-(2-hydroxyéthyl)-1-méthylquinoléin-2(1*H*)-one A une suspension de 5,9 g (23,4 mmoles) de 6-chloro-1-méthyl-1,2-dihydroquinoléine-4-acétate de méthyle dans 10 ml de méthanol et 100 ml de tétrahydrofurane sec, on ajoute 3,0 g (79 mmoles) de borohydrure de sodium puis on chauffe au reflux pendant 9 heures. Après refroidissement, on évapore les solvants sous vide et on reprend le résidu dans 400 ml de dichlorométhane et 100 ml d'acide chlorhydrique 3 N. On lave la phase organique à l'eau, on la sèche sur sulfate de sodium, on la filtre et on condense le filtrat. On purifie le produit brut par chromatoflash sur silice en éluant par un mélange méthanol/dichlorométhane (5:95).
   On obtient 5,9 g de l'alcool attendu.
   Rendement = 92 %
   Point de fusion = 169-170 °C
3.5. 6-chloro-4-(2-chloroéthyl)-1-méthylquinoléin-2(1*H*)-one A une suspension de 5,9 g (24,8 mmoles) de 6-chloro-4-(2-hydroxyéthyl)-1-méthylquinoléin-2(1*H*)-one dans 120 ml de chloroforme, deux gouttes de pyridine et deux gouttes de diméthylformamide, on ajoute goutte à goutte 5,5 ml (75 mmoles) de chlorure de thionyle. On chauffe le milieu réactionnel à un léger reflux pendant 2,5 heures puis on le traite comme décrit dans l'exemple 1.5.
   On obtient 5,4 g du produit attendu.
   Rendement = 86 %
   Point de fusion = 120-122 °C
3.6. chlorhydrate de 6-chloro-4-[2-[4-(4-fluorobenzoyl) pipéridin-1-yl]éthyl]-1-méthylquinoléin-2(1*H*)-one (1:1)
   On chauffe au reflux pendant 11 heures un mélange de 0,90 g (3,5 mmoles) de 6-chloro-4-(2-chloroéthyl)-1-méthylquinoléin-2(1*H*)-one, 0,71 g (4,0 mmoles) de 4-(4-fluorobenzoyl)pipéridine et 0,60 g 7,0 mmoles) de bicarbonate de sodium dans 15 ml d'acétonitrile. On évapore ensuite le milieu réactionnel à sec et on purifie le produit brut par chromatoflash sur silice en éluant par un mélange méthanol/dichlorométhane (4:96) contenant des traces d'ammoniaque.
   On obtient 0,86 g du produit attendu sous forme de base.
   Rendement = 62 %
   On prépare le chlorhydrate dans un mélange méthanol/acide chlorhydrique/éther.
   Point de fusion = 244 °C (décomposition)

### Exemple 4 (composé n° 5)

### chlorhydrate de 6-fluoro-1-méthyl-4-[2-[4-(thiéno-[3,2-c] pyridin-4-yl)pipérazin-1-yl]éthyl]quinoléin-2(1H)-one (2:1)

4.1. acide 3-(acétoxy)-5-[(4-fluorophényl)méthylamino]-5-oxopent-2-énoïque
   A une solution agitée de 6,64 g (53,1 mmoles) de N-méthyl-4-fluoroaniline dans 25 ml d'acide acétique pur, on ajoute par petites portions, 9,93 g (58,4 mmoles) de 4-(acéyloxy)-2*H*,3*H*-pyrane-2,6-dione. On agite le milieu réactionnel pendant 2 heures à 35 °C, on laisse refroidir à température ambiante et on dilue dans 500 ml d'eau glacée. On récupère le solide obtenu qui est essoré, lavé abondamment à l'eau et séché à l'étuve (40 °C) pendant 48 heures.
   On obtient 12,05 g du composé attendu sous forme de solide amorphe qui fond en dessous de 50 °C.
   Rendement = 76 %
4.2. acide 6-fluoro-1-méthyl-2-oxo-1,2-dihydroquinoléine-4-acétique
   On verse par petites portions 31,8 g (107 mmoles) d'acide 3-(acétyloxy)-5-[(4-fluorophényl)méthylamino]-5-oxopent-2-énoïque dans 130 ml d'acide sulfurique concentré à température ambiante sous forte agitation, puis on chauffe le milieu réactionnel à 90°C pendant 90 minutes. Après refroidissement, on verse cette solution dans un mélange de 500 g de glace et 500 ml d'eau. On essore le solide gris ainsi obtenu. On le lave à l'eau, puis on le triture dans l'éther et on le sèche pendant 24 heures à 40 °C.
   On obtient 11,37 g de produit.
   Point de fusion = 230 °C
   Rendement = 45 %
4.3. 6-fluoro-1-méthyl-2-oxo-1,2-dihydroquinoléine-4-acétate de méthyle
   On ajoute goutte à goutte, en 30 minutes environ, 16 ml (219 mmoles) de chlorure de thionyle à une suspension agitée de 11,37 g (49,38 mmoles) d'un mélange d'acide 6-fluoro-1-méthyl-2-oxo-1,2-dihydroquinoléine-4-acétique dans 120 ml de méthanol. On agite la nuit (13 heures) à température ambiante et on chasse le solvant sous vide. On dissout le résidu dans 400 ml de dichlorométhane, puis on le lave avec une solution aqueuse saturée en bicarbonate de sodium puis à l'eau. Après séchage sur sulfate de sodium, filtration et concentration du filtrat, on obtient 9,6 g du produit attendu.
   Rendement = 78 %
   Point de fusion = 134-135 °C
4.4. 6-fluoro-4-(2-hydroxyéthyl)-1-méthylquinoléin-2(1*H*)-one
   A une suspension de 8,0 g (32 mmoles) de 6-fluoro-1-méthyl-2-oxo-1,2-dihydroquinoléine-4-acétate de méthyle dans 100 ml de tétrahydrofurane sec, on ajoute 3,78 g (100 mmoles) de borohydrure de sodium et on chauffe au reflux pendant 20 heures. Après refroidissement à 5 °C, on ajoute, goutte à goutte, 2 ml de méthanol, on rajoute 3 g de borohydrure de sodium et on chauffe au reflux pendant 12 heures. On évapore les solvants sous vide et on reprend le résidu dans 400 ml de dichlorométhane et 150 ml d'acide chlorhydrique 2 N, on lave la phase organique à l'eau, puis on la sèche sur sulfate de sodium, on la filtre et on concentre le filtrat.
   On obtient 4,7 g de l'alcool attendu.
   Rendement = 66 %
   Point de fusion = 153-154 °C
4.5. 4-(2-chloroéthyl)-6-fluoro-1-méthylquinoléine-2(1*H*)-one A une suspension de 2,2 g (9,95 mmoles) de 6-fluoro-4-(2-hydroxyéthyl)-1-méthylquinoléin-2(1*H*)-one dans 100 ml de chloroforme, deux gouttes de pyridine et deux gouttes de diméthylformamide, on ajoute, goutte à goutte, 3 ml (41 mmoles) de chlorure de thionyle. On chauffe le milieu réactionnel à un léger reflux pendant 4,5 heures.
   Après refroidissement à température ambiante, on ajoute, goutte à goutte, 50 ml d'eau au milieu réactionnel et on laisse agiter 30 minutes. On récupère la phase organique, on la décante, on la lave à l'eau, on la sèche sur sulfate de magnésium et on filtre. On concentre le filtrat sous vide. On obtient 2,36 g du chloré attendu
   Rendement = 98 %
   Point de fusion = 141-142 °C
4.6. chlorhydrate de 6-fluoro-1-méthyl-4-[2-[4-[thiéno[3,2-*c*]pyridin-4-yl)pipérazin-1-yl]éthyl]quinoléin2(1*H*)-one (2:1)
   On ajoute 1,4 g (5,8 mmoles) de 4-(2-chloroéthyl)-6-fluoro-1-méthylquinoléine-2(1*H*)-one à un mélange de 1,3 g (5,9 mmoles) de 4-(pipérazin-1-yl)thiéno[3,2-*c*]pyridine et 0,50 g (5,95 mmoles) d'hydrogénocarbonate de sodium dans 20 ml d'acétonitrile et on chauffe le milieu réactionnel à 55-60 °C pendant 18 heures. On évapore le solvant et on reprend le résidu dans 100 ml de dichlorométhane. On le lave avec une solution aqueuse saturée en bicarbonate de sodium, puis à l'eau. On sèche la phase organique sur sulfate de sodium, on filtre et on condense le filtrat. On purifie le produit brut par chromatoflash sur silice en éluant par un mélange méthanol/dichlorométhane (5:95) contenant des traces d'ammoniaque. On obtient 0,70 g du produit attendu sous forme de base.
   Rendement = 27 %
   On dissout la base dans 10 ml de méthanol et on salifie avec un excès d'une solution d'acide chlorhydrique 2 N dans l'éther. On essore le précipité obtenu, on le recristallise dans du méthanol et on le sèche sous vide.
   On obtient 0,38 g du dichlorhydrate.
   Point de fusion = 280 °C (décomposition)

### Exemple 5 (composé n° 10)

### chlorhydrate de l'acide 7-fluoro-2-oxo-4-[2-[4-(thiéno [3,2-c]pyridin-4-yl)pipérazin-1-yl]éthyl]-1,2-dihydroquinoléine-1-acétique (2:1)

On ajoute, goutte à goutte, 2,9 ml d'une solution à 0,5 M de bromoacétate de tert-butyle dans le tétrahydrofurane à un mélange de 0,50 g (1,23 mmoles) de 7-fluoro-4-[2-[4-(thiéno [3,2-*c*]pyridin-4-yl)pipérazin-1-yl]éthyl]quinoléine-2(1*H*)-one (préparé à partir de 3-fluoroaniline suivant la méthode décrite dans l'exemple 4), 0,10 g (1,79 mmoles) d'hydroxyde de potassium fraîchement moulu et 0,12 g (0,37 mmole) de bromure de tétrabutylammonium dans 20 ml de tétrahydrofurane à 0-5 °C. Après 30 minutes à 0-5 °C, on laisse remonter la température à l'ambiante et on continue l'agitation pendant 6 heures. On évapore le solvant sous vide et on reprend le résidu dans 100 ml de dichlorométhane, on lave à l'eau, on sèche la phase organique sur sulfate de sodium et on condense. On purifie le produit brut par chromatoflash sur silice en éluant par un mélange méthanol/dichlorométhane (5:95) contenant des traces d'ammoniaque et on obtient 0,48 g de *N*-acétate de *tert*-butyle sous forme d'huile épaisse incolore.
Rendement = 75 %
A cette huile, on ajoute 50 ml d'une solution 3 N d'acide chlorhydrique dans l'acétate d'éthyle et on agite à température ambiante pendant 4 heures. On évapore à sec et on triture le solide blanc obtenu à l'éther et on le sèche sous vide.
On obtient 0,47 g de l'acide attendu sous forme de dichlorhydrate.
Rendement = 87 %
Point de fusion = 218-220 °C (décomposition)

### Exemple 6 (composé n° 12)

### chlorhydrate de 7-fluoro-2-oxo-4-[2-[4-(thiéno[3,2-c]pyridin-4-yl)pipérazin-1-yl]éthyl]-1,2-dihydroquinoléine-1-acétamide (2:1)

On ajoute goutte à goutte 3,9 ml d'une solution à 0,5 M de bromoacétamide dans le tétrahydrofurane à un mélange agité de 0,53 g (1,3 mmoles) de 7-fluoro-4-[2-[4-(thiéno[3,2-*c*] pyridin-4-yl)éthyl]quinoléine-2(1*H*)-one, 0,1 g (1,79 mmoles) d'hydroxyde de potassium moulu et 0,13 g (0,4 mmole) de bromure de tétrabutylammonium dans 25 ml de tétrahydrofurane à 0-5°C. Après 30 minutes, on laisse monter la température à l'ambiante et on agite à cette température pendant 20 heures. On évapore le milieu réactionnel à sec sous vide et on reprend le résidu dans 100 ml de dichlorométhane. On lave cette solution à l'eau. On sèche la phase organique sur sulfate de magnésium et on la concentre. On triture le produit brut dans un mélange éther/dichlorométhane (1:3) puis on essore le solide et on le purifie par chromatographie sur silice en éluant par un mélange méthanol/acétate d'éthyle (10:90) puis par un mélange méthanol/dichlorométhane (10:90) contenant des traces d'ammoniaque.
On obtient 0,303 g d'un solide blanc que l'on transforme en dichlorhydrate dans un mélange acide chlorhydrique/éther 2 M, méthanol.
On obtient 0,32 g de dichlorhydrate.
Point de fusion = 280 °C (décomposition)

### Exemple 7 (composé n° 20)

### chlorhydrate de 1-méthyl-2-oxo-4-[2-[4-(thiéno[3,2-c] pyridin-4-yl)pipérazin-1-yl]éthyl]-1,2-dihydroquinoléine-6-carbonitrile (2:1)

7.1. 6-cyano-1-méthyl-2-oxo-1,2-dihydroquinoléine-4-acétate de méthyle
   A une solution de 0,50 g (1,4 mmoles) de 6-iodo-1-méthyl-2-oxo-1,2-dihydroquinoléin-4-acétate de méthyle (préparé à partir de la *N*-méthyl-4-iodoaniline suivant la méthode décrite dans l'exemple 1) dans 6 ml de triéthylamine anhydre, on ajoute 1,1 ml de cyanure de triméthylsilyle (8,4 mmoles) suivi de 0,15 g (0,13 mmole) de palladium tétrakistriphénylphosphine. On chauffe ensuite le milieu réactionnel pendant 4 heures sous atmosphère d'azote au reflux. Après refroidissement à température ambiante, on verse le milieu dans 60 ml de toluène et 60 ml d'eau. On lave la phase organique à l'eau et on réextrait la phase aqueuse initiale au dichlorométhane. On réunit les phases organiques, on les sèche sur sulfate de sodium et on les concentre sous vide. On purifie le résidu par chromatoflash sur silice en éluant par un mélange méthanol/dichlorométhane (5:95).
   On obtient 0,313 g du nitrile attendu.
   Rendement = 87 %
   Point de fusion = 202-203 °C
7.2. 6-cyano-4-(2-hydroxyéthyl)-1-méthylquinoléin-2(1*H*)-one
   7.2.1. acide 6-cyano-1-méthyl-2-oxo-1,2-dihydroquinoléine-4-acétique
      A 1,21 g (4,7 mmoles) de 6-cyano-1-méthyl-2-oxo-1,2-dihydroquinoléine-4-acétate de méthyle dans 10 ml de méthanol à 0-5 °C, on ajoute, goutte à goutte, 10,4 ml d'une solution d'hydroxyde de lithium 0,5 N (5,2 mmoles). On laisse remonter la température à l'ambiante et on agite pendant 2 heures. On verse le milieu réactionnel dans 250 ml d'eau glacée et on acidifie à pH 2-3 avec de l'acide chlorhydrique 4 N. On essore le précipité blanc formé, on le lave à l'eau, puis on le sèche sous vide à 40 °C.
      On obtient 0,85 g du produit attendu.
      Rendement = 75 %
      Point de fusion = 238 °C
   7.2.2. 6-cyano-4-(2-hydroxyéthyl)-1-méthylquinoléin-2(1*H*)-one
      A une suspension de 0,365 g (1,51 mmoles) d'acide 6-cyano-1-méthyl-2-oxo-1,2-dihydroquinoléine-4-acétique dans 10 ml de tétrahydrofurane à -10 °C, on ajoute 0,22 ml (1,58 mmoles) de triéthylamine puis 0,16 ml (1,6 mmoles) de chloroformiate d'éthyle, goutte à goutte. Après agitation à -10 °C pendant 45 minutes, on filtre le milieu réactionnel et on rince les solides avec 3 x 8 ml de tétrahydrofurane. Au filtrat à 5-10 °C, on ajoute 0,25 g (6,61 mmoles) de borohydrure de sodium puis 0,94 ml de méthanol. Après agitation à 5-10 °C pendant 2 heures, on ajoute 13 ml d'une solution aqueuse d'acide chlorhydrique 1 N. On extrait au dichlorométhane puis à l'acétate d'éthyle. On sèche les phases organiques sur sulfate de sodium puis on concentre sous vide.
      On obtient 0,315 g de produit.
      Rendement = 92 %
      Point de fusion = 231-233 °C
7.3. 4-(2-bromoéthyl)-6-cyano-1-méthylquinoléin-2(1*H*)-one
   A 0,48 g (1,14 mmoles) de dibromotriphénylphosphorane dans 14 ml de dichlorométhane à température ambiante, on ajoute par petites quantités 0,24 g (1,05 mmoles) de 6-cyano-4-(2-hydroxyéthyl)-1-méthylquinoléin-2(1*H*)-one. Après 75 minutes d'agitation à température ambiante, on verse le milieu réactionnel dans 200 ml de dichlorométhane et on lave à l'eau. On sèche la phase organique sur sulfate de sodium, on filtre et on condense sous vide. On triture le résidu blanc dans de l'éther diéthylique. On reprend le solide obtenu dans un minium de dichlorométhane, on filtre rapidement à travers une couche de silice en éluant avec de l'éther et on évapore le filtrat.
   On obtient 0,20 g de produit utilisé tel quel.
   Rendement = 65 %
7.4. chlorhydrate de 1-méthyl-2-oxo-4-[2-[4-(thiéno [3,2-*c*]pyridin-4-yl)pipérazin-1-yl]éthyl]quinoléin-2 (1*H*)-one (2:1)
   On chauffe à 55 °C pendant 36 heures un mélange de 0,19 g (0,65 mmole) de 4-(2-bromoéthyl)-6-cyano-1-méthylquinoléin-2 (1*H*)-one, 0,15 g (0,65 mmole) de 4-(pipérazin-1-yl)thiéno [3,2-c]pyridine et de 0,09 g (0,11 mmole) de bicarbonate de sodium dans 10 ml d'acétonitrile. On évapore à sec le milieu réactionnel, on reprend le résidu dans 100 ml de chloroforme et on lave à l'eau. On sèche la phase organique sur sulfate de sodium, on concentre et on purifie le produit brut par chromatoflash sur silice en éluant par un mélange méthanol/dichlorométhane (1:9) contenant des traces d'ammoniaque.
   On obtient 0,211 g de base sous forme d'une huile incolore. Rendement = 48 %
   On prépare le dichlorhydrate dans un mélange méthanol/ éther/acide chlorhydrique 2 N.
   On obtient 0,182 g de produit sous forme de dichlorhydrate.
   Point de fusion = 200 °C (décomposition)

### Exemple 8 (composé n° 17)

### chlorhydrate de 6-hydroxy-1-méthyl-4-[2-[4-(thiéno[3,2-c] pyridin-4-yl)pipérazin-1-yl]éthyl]quinoléin-2(1H)-one (2:1)

On ajoute 0,47 g (1,08 mmoles) de 6-méthoxy-1-méthyl-4-[2-[4-(thiéno[3,2-*c*]pyridin-4-yl)pipérazin-1-yl]éthyl]quinoléin2(1*H*)-one (obtenue à partir de 6-méthoxy-1-méthyl-2-oxo-1,2-dihydroquinoléine-4-acétate de méthyle suivant l'exemple 1) à 25 ml d'acide bromhydrique à 48 % et on porte au reflux pendant 3 heures. Après refroidissement, on filtre le précipité gris, on le lave à l'eau froide et cn le sèche sous vide à 40 °C. On obtient 0,444 g du produit sous forme de dibromhydrate.
Rendement = 71%
On reprend 0,14 g (0,24 mmole) de ce produit dans 20 ml d'acide chlorhydrique 3,7 N dans le méthanol anhydre et on agite à température ambiante pendant 3 heures. On essore le précipité, on le rince avec de l'éther diéthylique et on le sèche à l'étuve.
On obtient 0,112 g du produit attendu.
Rendement = 95 %
Point de fusion = 227 °C (décomposition)

### Exemple 9 (composé n° 18)

### chlorhydrate de 6-nitro-4-[2-[4-(thiéno[3,2-c]pyridin-4-yl)pipérazin-1-yl]éthyl]quinoléin-2(1H)-one (2:1)

9.1. 4-(2-chloroéthyl)-6-nitroquinoléin-2(1*H*)-one A un mélange de 120 ml d'acide nitrique à 65 % et 80 ml d'acide sulfurique concentré refroidi à 5 °C, on ajoute par petites quantités, 20,0 g (96,4 mmoles) de 4-(2-chloroéthyl) quinoléin-2(1*H*)-one et on chauffe le mélange pendant 2 heures à 45 °C. On verse le milieu réactionnel sur 600 ml d'eau glacée, on essore le précipité jaune pâle, on le rince à l'eau et on le sèche sous vide.
   On obtient 22,5 g de produit attendu.
   Rendement = 92 %
   Point de fusion = 239-237 °C
9.2. chlorhydrate de 6-nitro-4-[2-[4-(thiéno[3,2-*c*]pyridin-4-yl)pipérazin-1-yl]éthyl]quinoléin-2(1*H*)-one (2:1)
   On chauffe à 50 °C pendant 20 heures un mélange de 1 g (3,96 mmoles) de 4-(2-chloroéthyl)-6-nitroquinoléin-2(1*H*)-one, 0,87 g (4 mmoles) de 4-(pipérazin-1-yl)thiéno[3,2-*c*] pyridine et 0,5 g (5,95 mmoles) de bicarbonate de sodium dans 10 ml de diméthylformamide. Ensuite on filtre le résidu et on le lave à l'eau, on ajoute 200 ml d'eau au filtrat, on essore le précipité formé et on le sèche sous vide.
   On obtient 1,28 g du produit attendu sous forme de base.
   Rendement = 74 %
   On prépare le chlorhydrate dans un mélange méthanol/éther/ acide chlorhydrique.
   Point de fusion = 242 °C (décomposition)

### Exemple 10 (composé n° 16)

### chlorhydrate de 6-amino-4-[2-[4-(thiéno[3,2-c]pyridin-4-yl)pipérazin-1-yl]éthyl]quinoléin-2(1H)-one (3:1)

10.1. chlorhydrate de 6-amino-4-(2-chloroéthyl)quinoléin-2(1*H*)-one (1:1)
   A une suspension de 3,5 g (13,8 mmoles) de 4-(2-chloroéthyl)-6-nitroquinoléin-2(1*H*)-one dans 300 ml de méthanol à la température ambiante, on ajoute 0,70 g de palladium sur charbon à 5 % et on agite sous une pression de 8 psi (0,06 MPa) d'hydrogène pendant 3 heures. On filtre le catalyseur et on condense le filtrat.
   On obtient 2,97 g du produit sous forme de base.
   On prépare le chlorhydrate dans un mélange méthanol/éther/ acide chlorhydrique.
   Point de fusion > 290 °C
10.2. chlorhydrate de 6-amino-4-[2-[4-(thiéno[3,2-*c*] pyridin-4-yl)pipérazin-1-yl]éthyl]quinoléin-2 (1*H*)-one (3:1)
   On chauffe à 60 °C pendant 24 heures un mélange de 0,35 g (1,35 mmoles) de chlorhydrate de 6-amino-4-(2-chloroéthyl) quinoléin-2(1*H*)-one, 0,33 g (1,5 mmoles) de 4-(pipérazin-1-yl)thiéno[3,2-*c*] pyridine et 0,17 g (2 mmoles) de bicarbonate de sodium dans 10 ml de diméthylformamide. Après refroidissement à la température ambiante, on dilue le milieu réactionnel dans 50 ml d'eau et on extrait le produit brut au chloroforme. On sèche la phase organique sur sulfate de sodium et on la concentre. On purifie le produit brut par chromatoflash sur silice en éluant d'abord par un mélange méthanol/acétate d'éthyle (6,5:93,5) contenant des traces de triéthylamine puis par un mélange méthanol/dichlorométhane (6,5:93,5) comprenant des traces d'ammoniaque.
   On obtient 0,14 g de produit sous forme de base.
   Rendement = 26 %
   On prépare ensuite le trichlorhydrate dans des conditions classiques.
   Point de fusion = 233 °C (décomposition)

### Exemple 11 (composé n° 33)

### chlorhydrate de 6-acétylamino-4-[2-[4-(thiéno[3,2-c]pyridin-4-yl)pipérazin-1-yl]éthyl]quinoléin-2(1H)-one (2:1)

11.1. chlorhydrate de 6-acétylamino-4-(2-chloroéthyl) quinoléin-2(1*H*)-one (1:1)
   A une suspension de 1,0 g (4,49 mmoles) de 6-amino-4-(2-chloroéthyl)quinoléin-2(1*H*)-one dans 50 ml de chloroforme à la température ambiante, on ajoute 0,75 ml (5,39 mmoles) de triéthylamine puis 0,35 ml (4,9 mmoles) de chlorure d'acétyle. On agite le mélange pendant 16 heures puis on le dilue dans 200 ml de chloroforme. On lave la suspension avec une solution aqueuse d'acide chlorhydrique 1 N et on essore le précipité.
   On obtient 0,72 g du produit attendu.
   Rendement = 60 %
11.2. chlorhydrate de 6-acétylamino-4-[2-[4-(thiéno[3,2-*c*] pyridin-4-yl)pipérazin-1-yl]éthyl]quinoléin-2(1*H*)-one (2:1)
   On chauffe à 60 °C pendant 24 heures un mélange de 0,35 g (1,32 mmoles) de chlorhydrate de 6-acétylamino-4-(2-chloroéthyl)quinoléin-2(1*H*)-one, 0,38 g (1,75 mmoles) de 4-(pipérazin-1-yl)thiéno[3,2-c]pyridine et 0,17 g (2 mmoles) de bicarbonate de sodium dans 10 ml de diméthylformamide. Après refroidissement à la température ambiante, on dilue le milieu réactionnel dans 100 ml d'eau et on laissse au repos pendant une nuit à 5 °C. On essore le solide formé et on le sèche sous vide. On purifie le produit brut par chromatoflash sur silice en éluant d'abord par un mélange méthanol/acétate d'éthyle (5:95) puis par un mélange méthanol/dichlorométhane (10:90) comprenant des traces d'ammoniaque.
   On obtient 0,20 g de produit sous forme de base.
   Rendement = 34 %
   On prépare ensuite le dichlorhydrate dans des conditions classiques.
   Point de fusion = 225 °C (décomposition)

### Légende du tableau :

- dans la colonne 'Sel':
   HCl représente un chlorhydrate,
   les rapports (x:y) correspondent au rapport (acide:base),
   l'absence de toute mention signifie que le composé est sous forme de base.
- dans la colonne 'Point de fusion' :
   "(d)" correspond à une fusion avec décomposition.

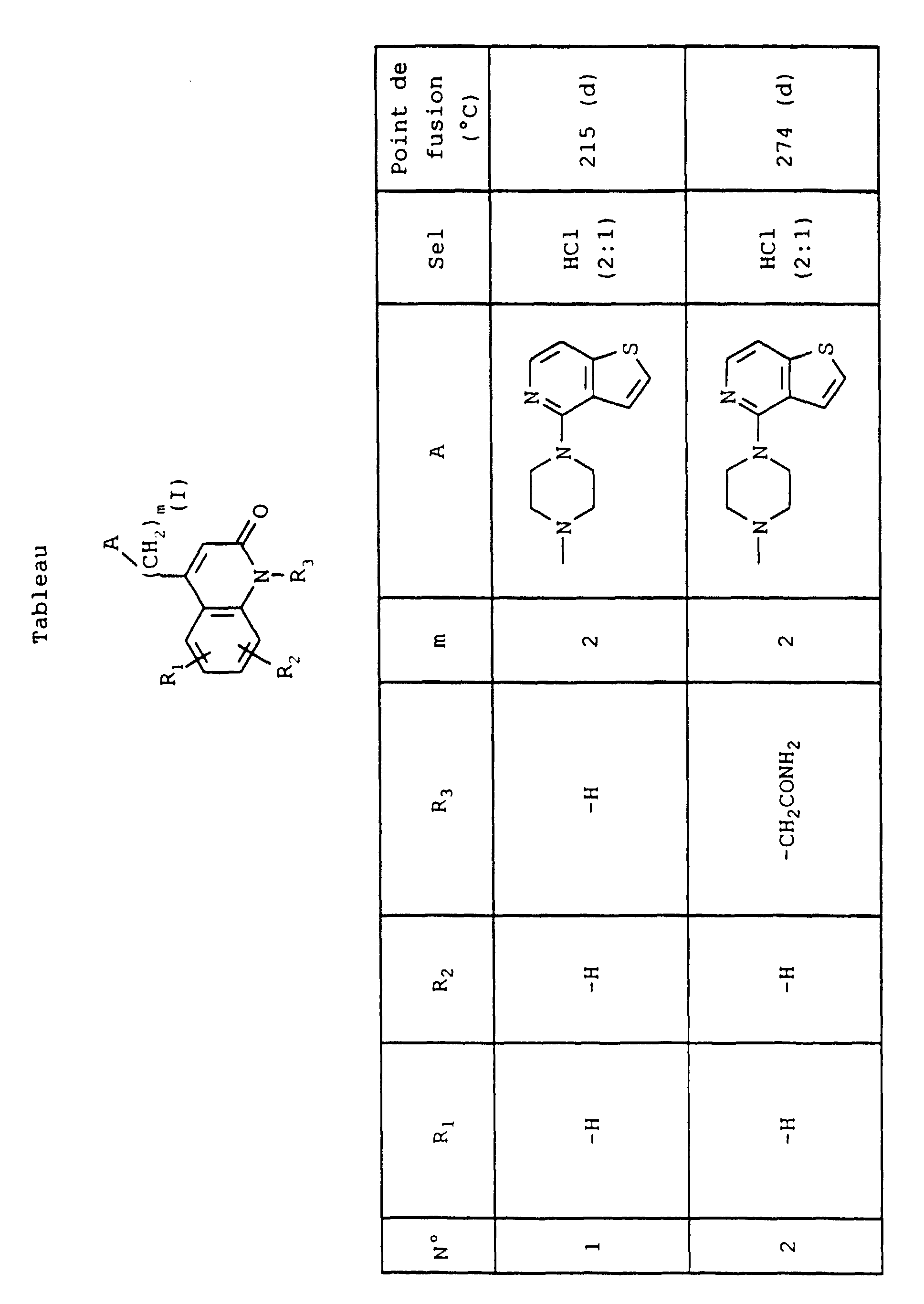

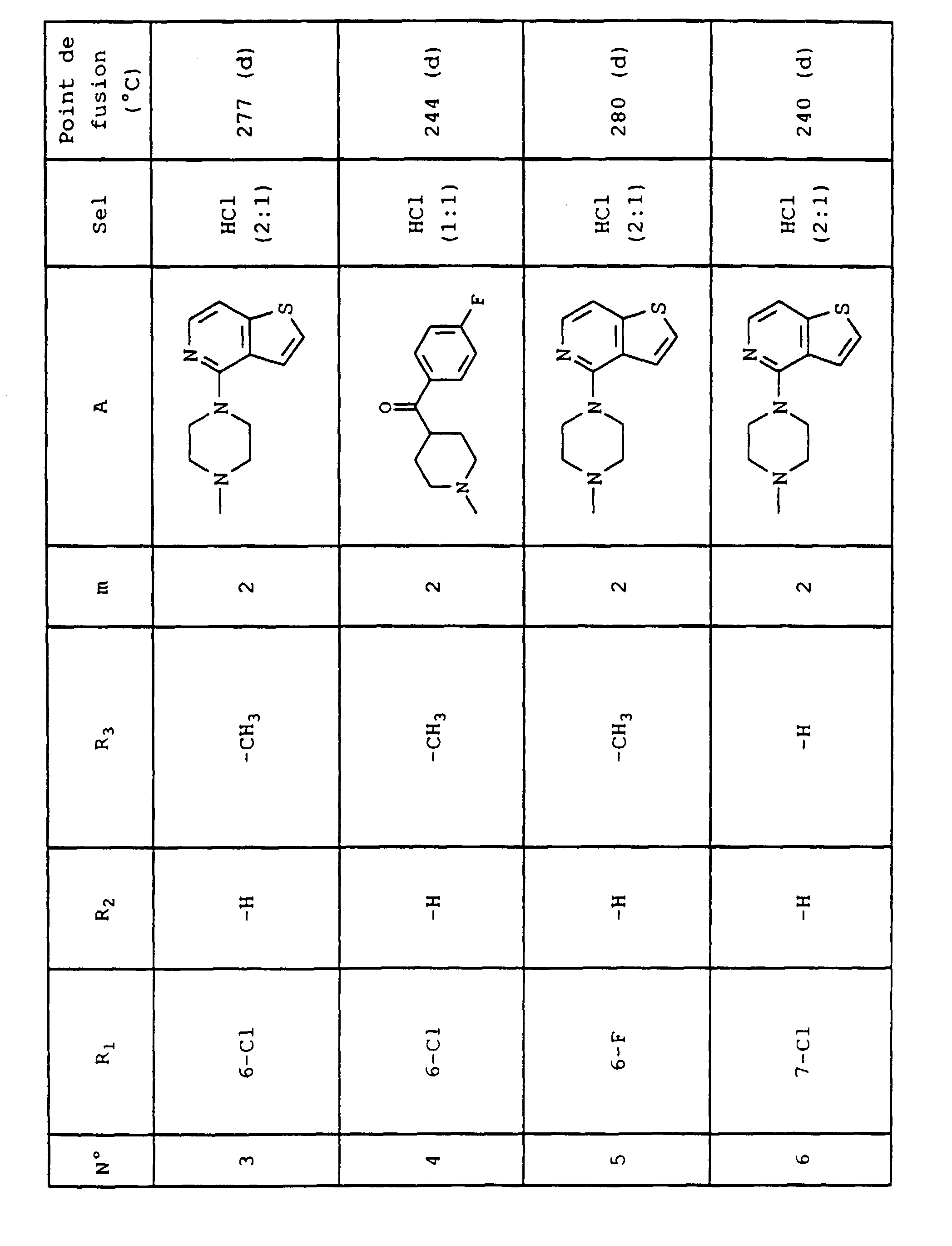

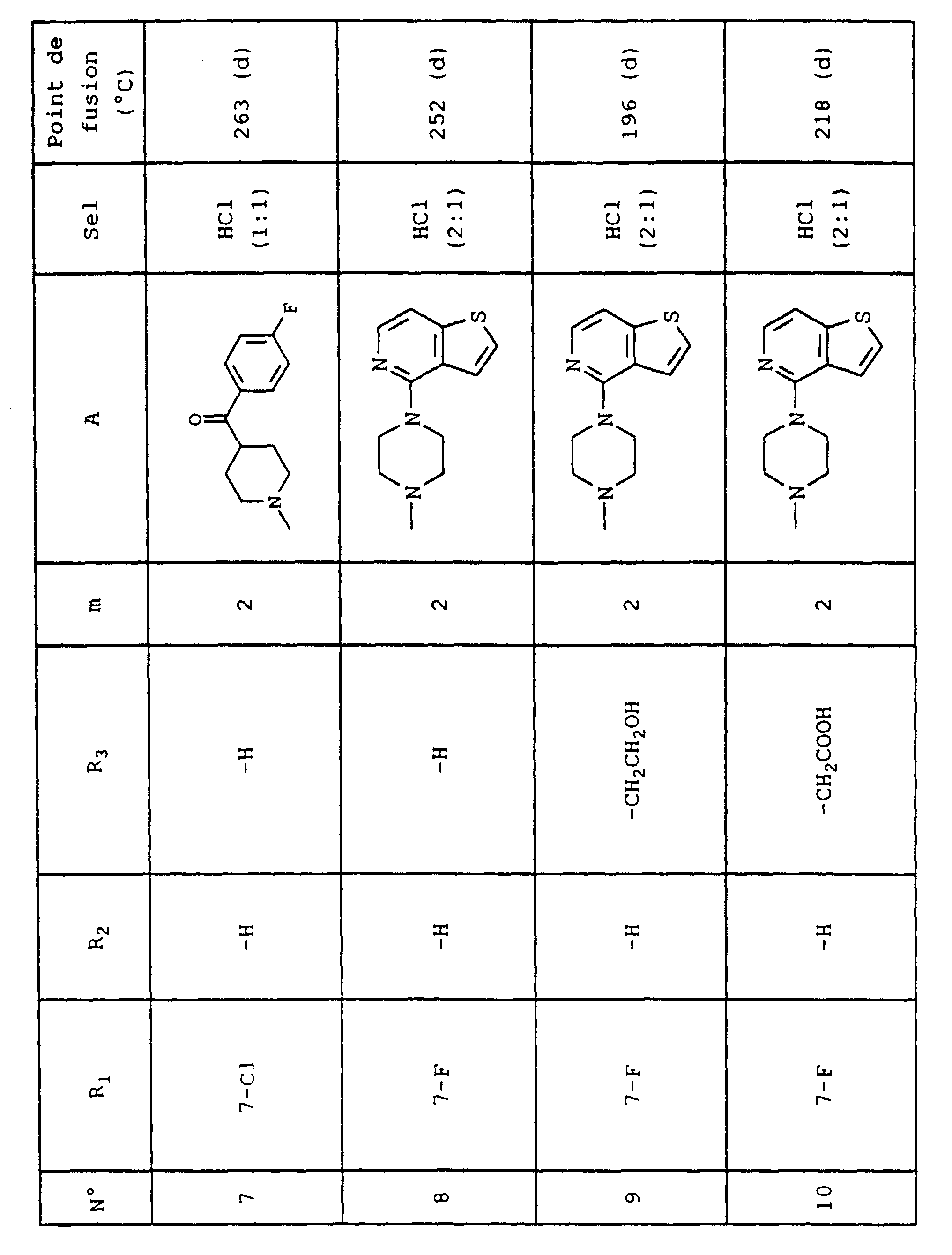

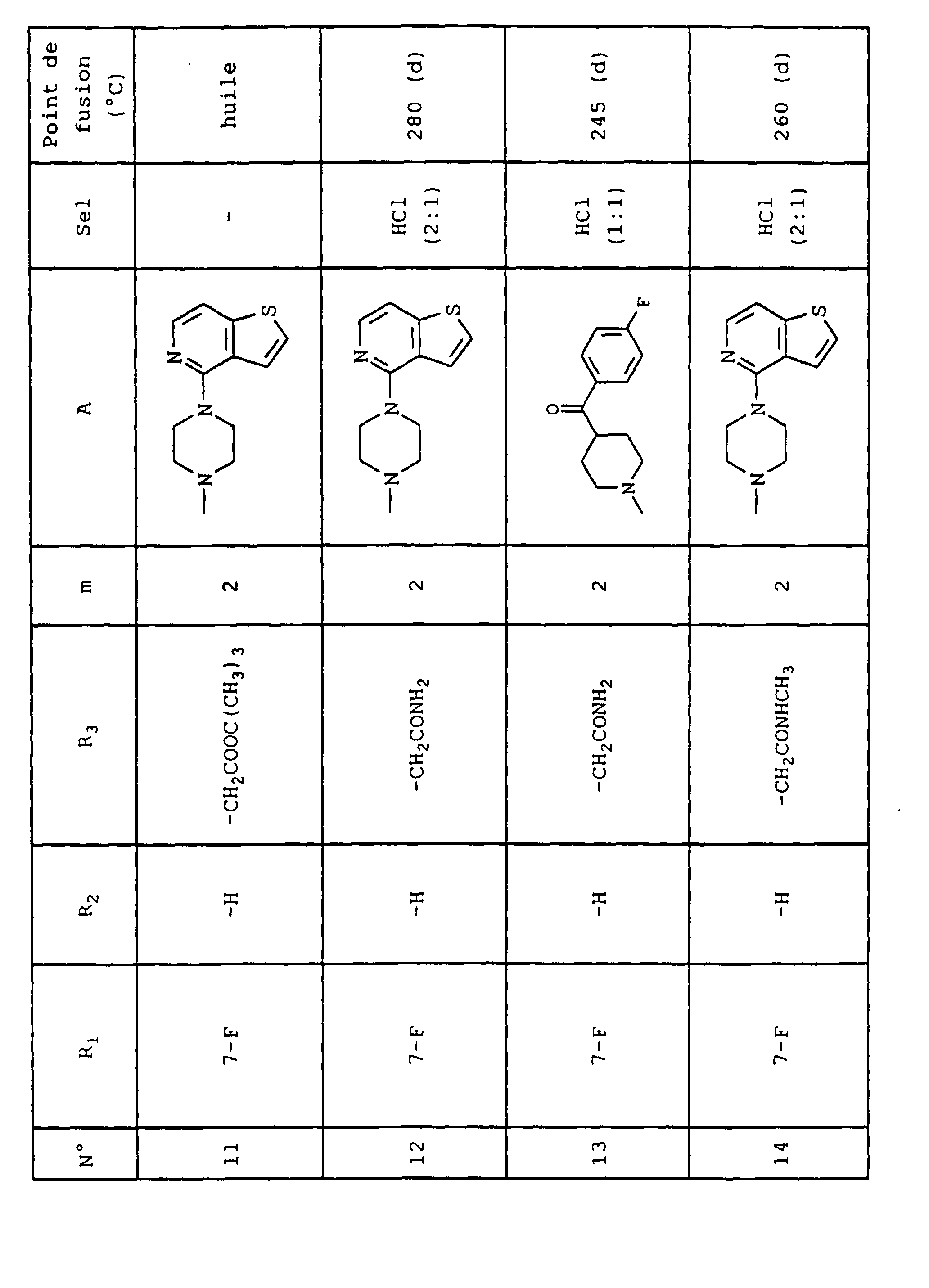

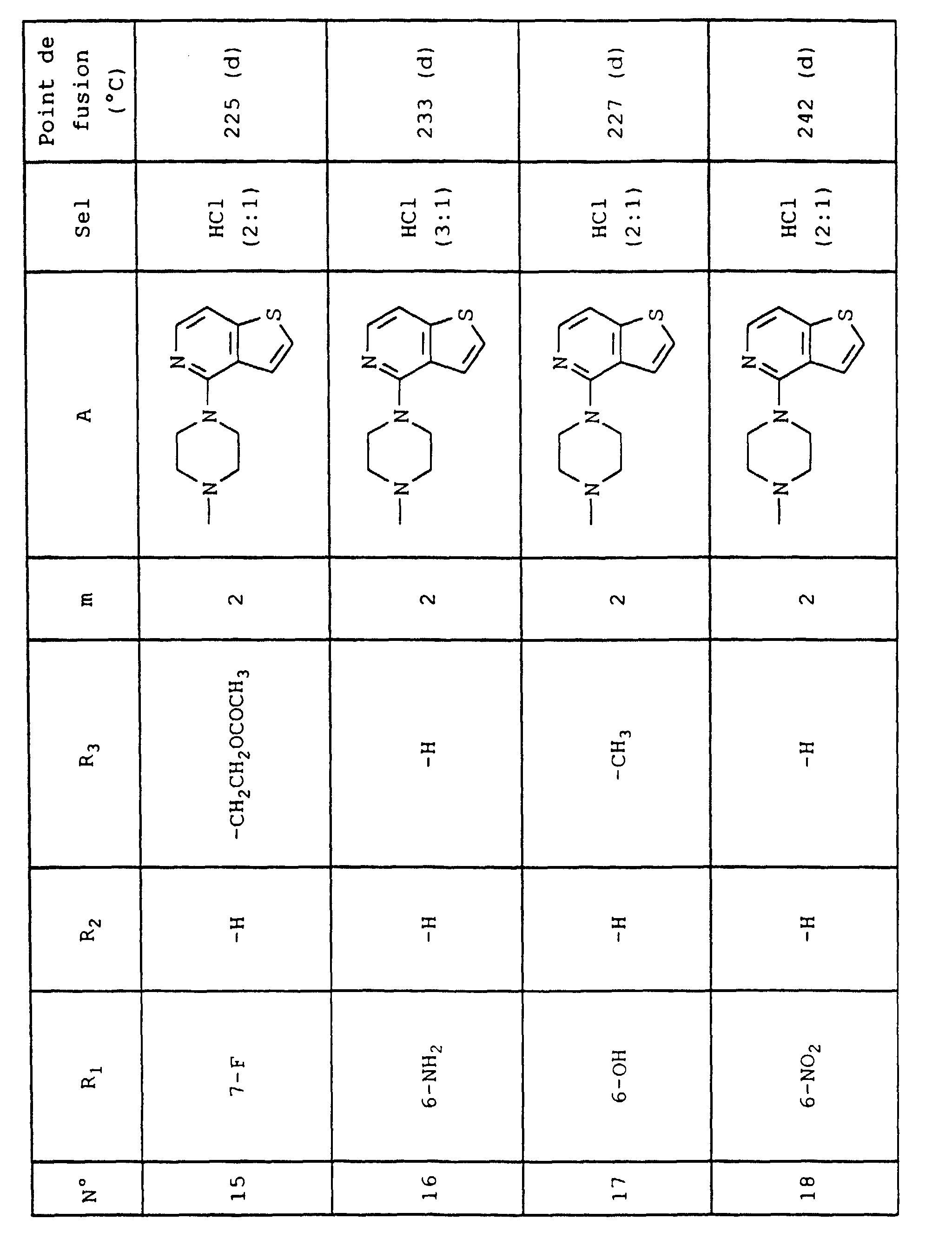

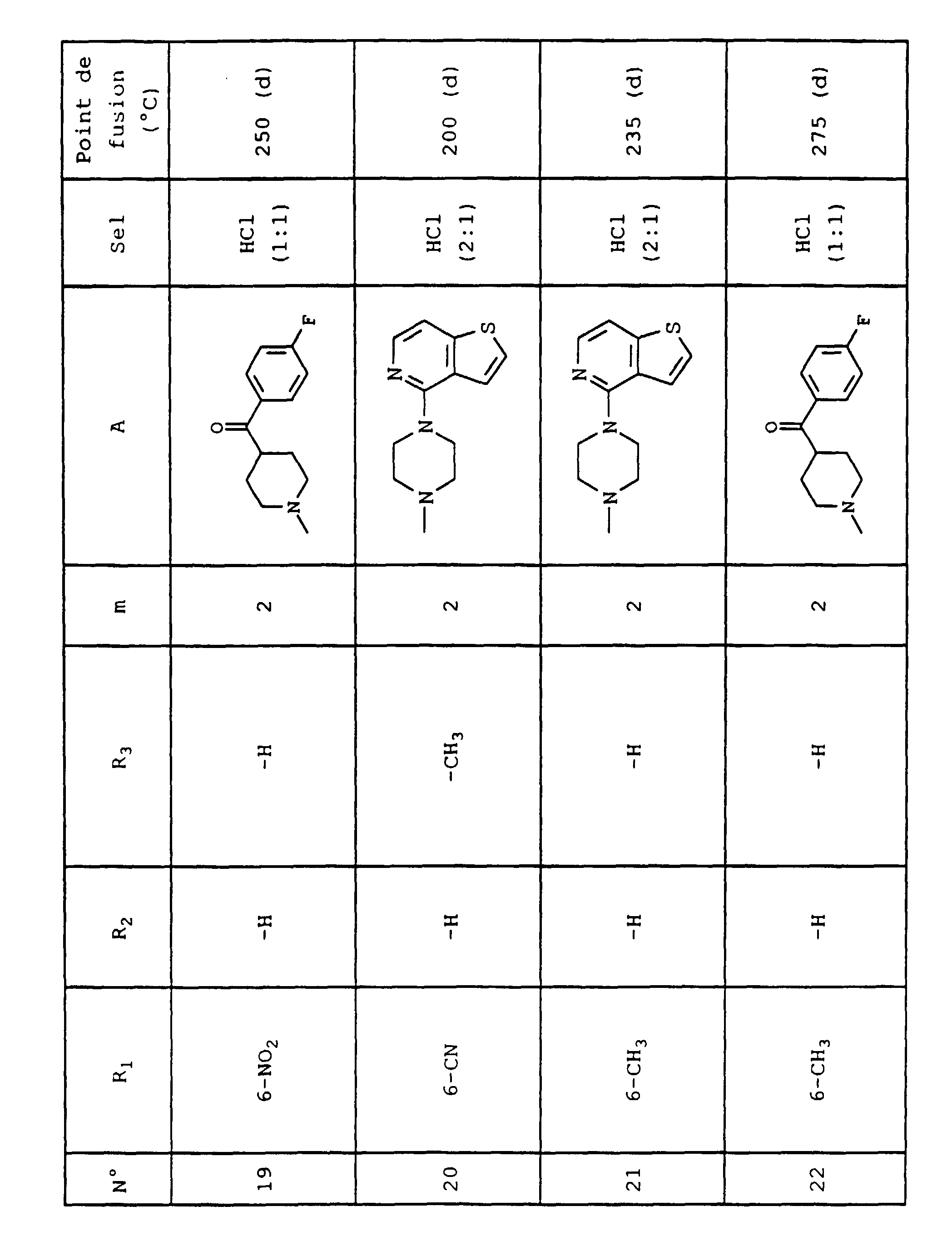

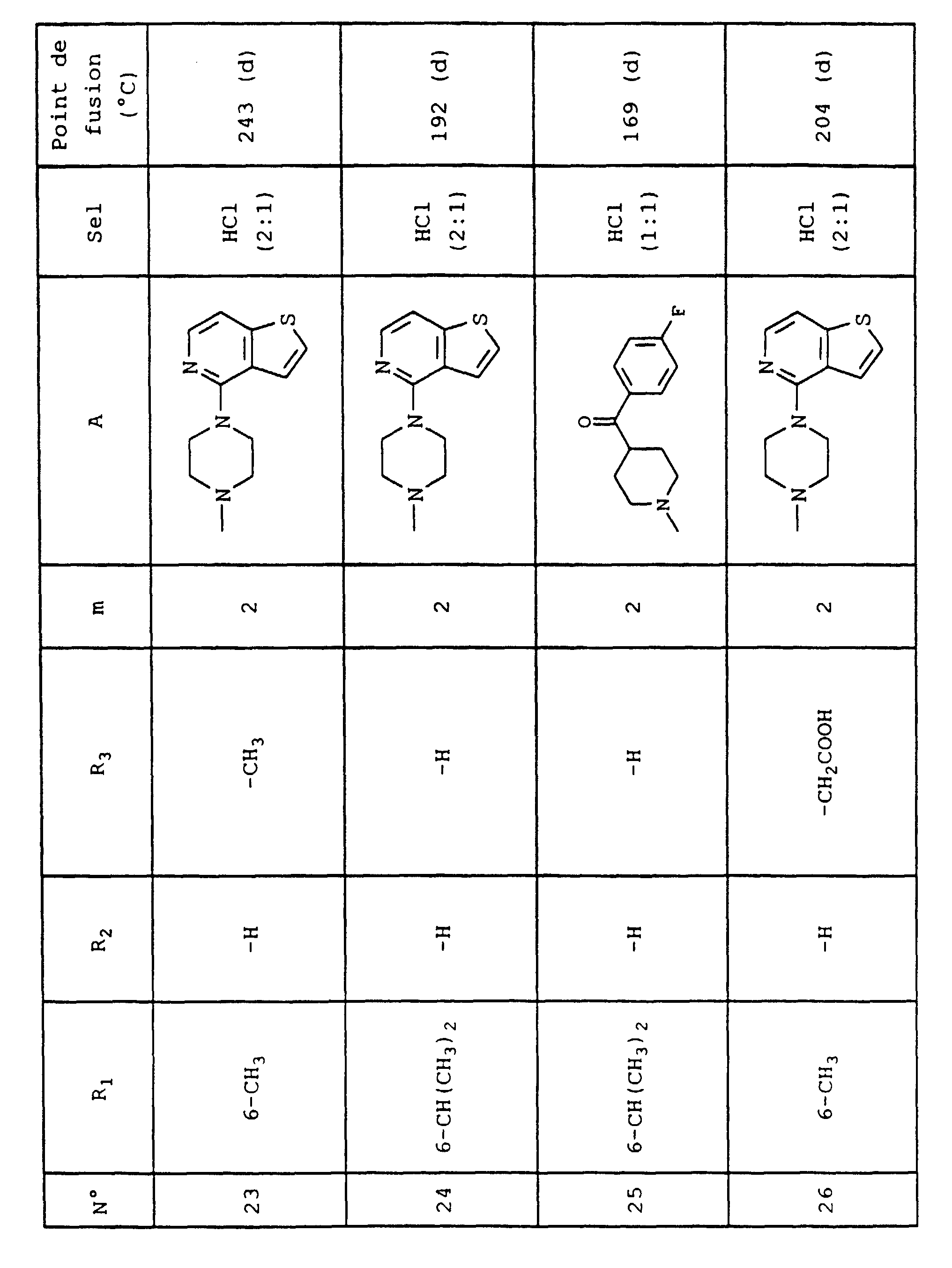

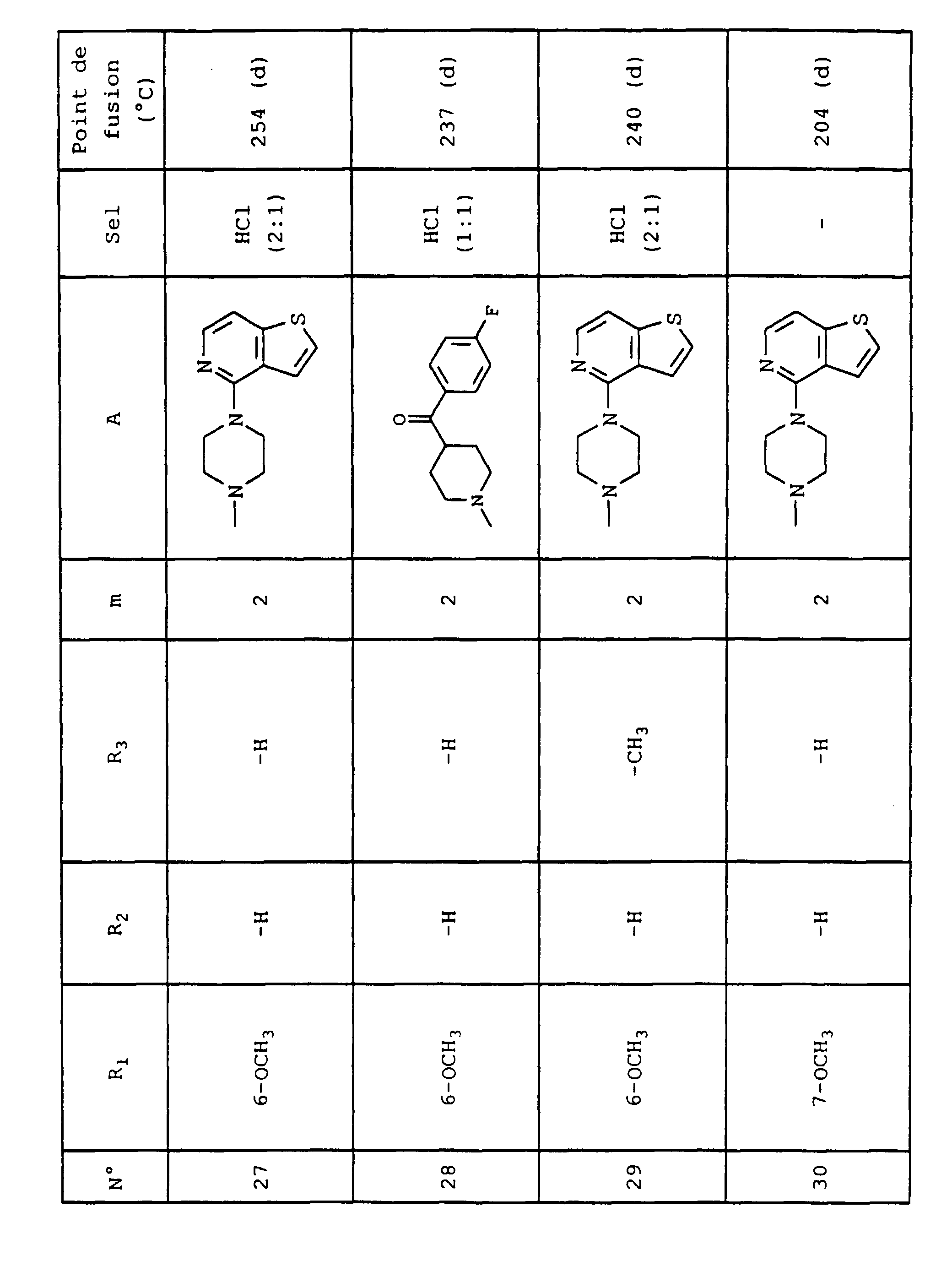

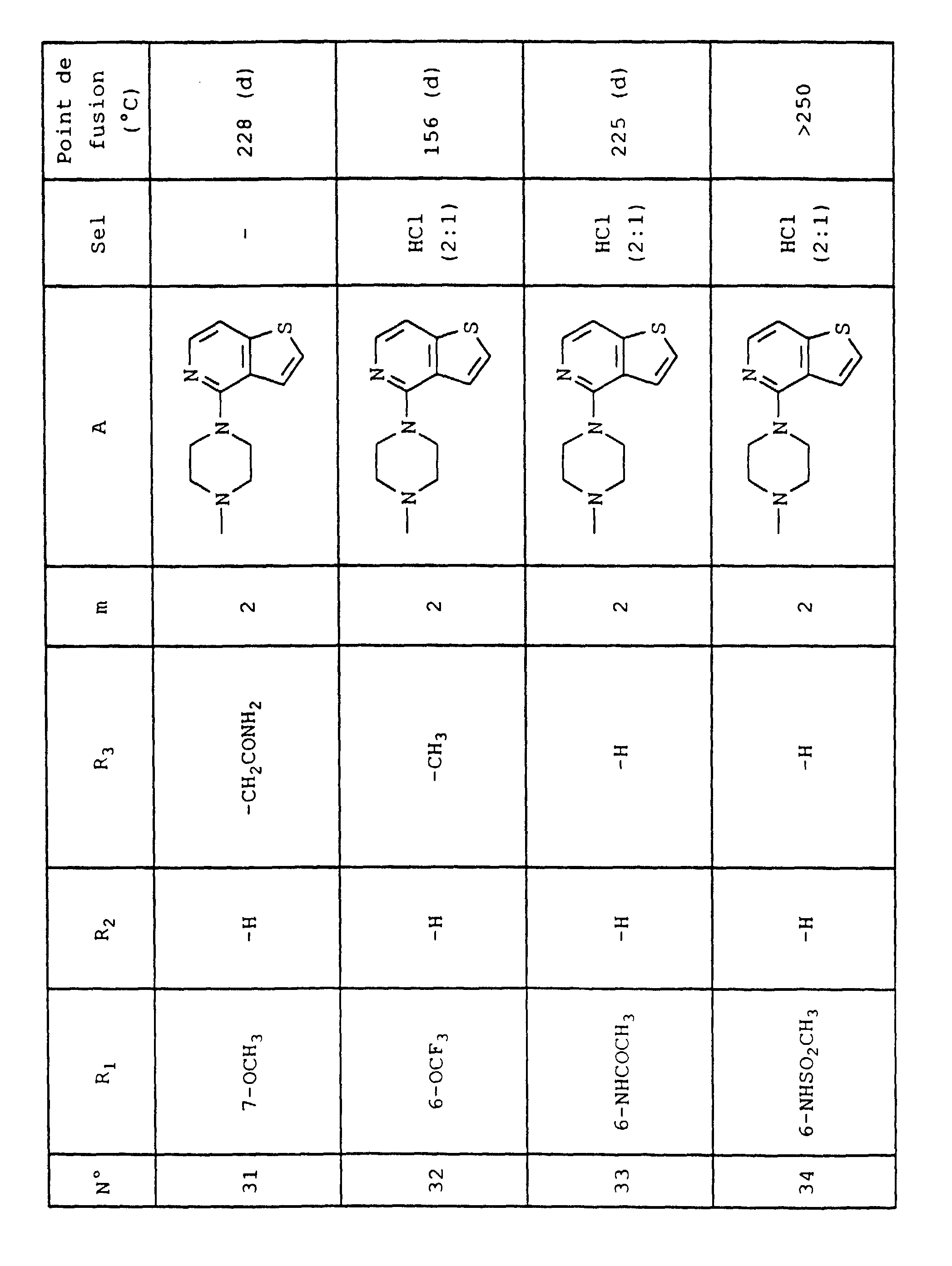

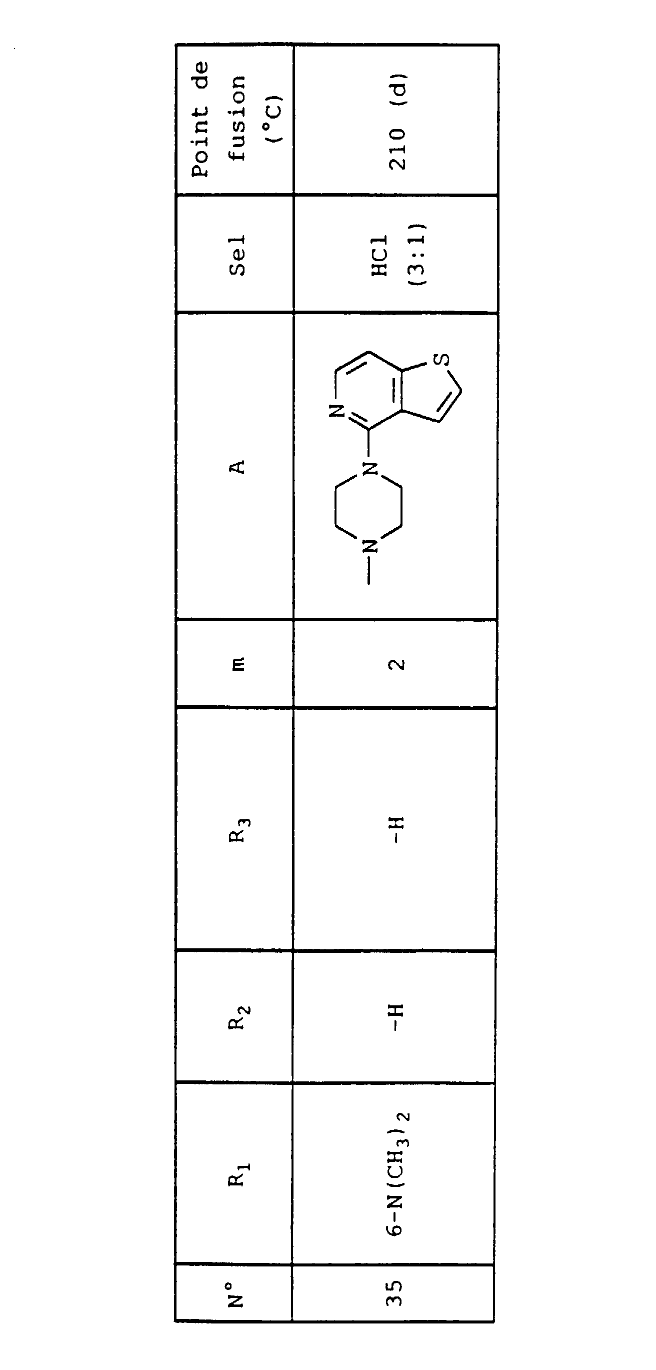

Les composés de l'invention ont fait l'objet d'études pharmacologiques qui ont mis en évidence leurs propriétés antagonistes de la sérotonine et leur intérêt comme substances à activité thérapeutique.

Ainsi, les composés de l'invention ont été soumis à un test d'inhibition de l'effet vasopresseur de la sérotonine. On utilise des rats mâles (Sprague-Dawley, Charles River France) pesant 250 à 300 g, que l'on anesthésie au pentobarbital sodique (60 mg/kg/i.p.) et que l'on maintient sous respiration artificielle (Respirateur Harvard ™-fréquence de respiration de 70 ml par minute, volume d'air 1 ml par 100 g de poids corporel). On amyèle les animaux à l'aide d'une tige métallique, introduite par l'orbite de l'oeil droit descendue le long de la colonne vertébrale. On sectionne les nerfs vagues droit et gauche (bivagotomie), on ligature l'artère carotide droite, l'artère carotide gauche étant cathétérisée afin de mesurer la pression artérielle à l'aide d'une cellule de pression (type Statham™ P23Db). On cathétérise une veine fémorale en vue de l'administration de divers composés. On mesure les augmentations de pression artérielle moyenne induite par la sérotonine administrée par voie intraveineuse à la dose de 30 µg/kg. Les composés de l'invention ou le véhicule sont administrés 5 minutes (pour les études par voie i.v.) ou 75 minutes (pour les études par voie orale) avant l'administration de la sérotonine. Les composés de l'invention sont administrés à des doses allant de 0.001 à 10 mg/kg. Le pourcentage d'inhibition de la réponse contrôle à la sérotonine est utilisé pour apprécier le potentiel antagoniste à la sérotonine des composés de l'invention.

Les composés de l'invention ont également été testés dans un modèle de vasoconstriction au Sumatriptan de la veine saphène isolée de chien (activité antagoniste au niveau du récepteur 5-HT₁₋ₗᵢₖₑ, selon HUMPHREY et al. dans *Br. J. Pharmacol.* 1988, 94, 1123).
Des veines saphènes de chiens Beagles ou Anglopoitevins sont prélevées sous anesthésie au pentobarbital administré en injection intraveineuse. Le vaisseau est découpé en hélice de 0,4 cm de large puis divisé en segments de 0,5 cm de longueur. Chaque fragment, monté entre deux serre-fines, est placé dans une cuve à organes isolés contenant 20 ml d'une solution physiologique de Krebs de composition suivante (mM) : NaCl 118; KCl 4,7; MgCl₂ 1,2; CaCl₂ 2,6; NaHCO₃ 25; Glucose 11,1 ; acide ascorbique 0,11. L'organe, maintenu à 37 °C sous un courant de carbogène (95 %O₂-5 % CO₂) à pH 7,4 est relié à un capteur isométrique Hugo Sachs type 351 sous une tension basale de 2 g et connecté à un polygraphe Gould 2400S permettant l'enregistrement des variations tensionnelles. L'acquisition des données est automatisée par système micro-informatique. Après un repos de 90 minutes entrecoupé de fréquents rinçages pendant lequel la tension basale est réajustée, l'organe est stimulé par 3 µM de noradrénaline afin de vérifier sa viabilité. On construit alors une courbe concentration-réponse contractile au Sumatriptan de façon cumulative entre 10 nM et 10 µM. Lorsque la contraction maximale est obtenue (plateau de l'effet à deux concentrations consécutives de Sumatriptan), on rince abondamment la préparation en intercalant des périodes de repos pour permettre à l'organe de revenir à la tension initiale. Le composé à étudier est alors ajouté dans le bain à organes 15 minutes avant qu'une deuxième courbe concentration-réponse au Sumatriptan soit construite. Les réponses de contraction obtenues en présence du composé sont exprimées en pourcentage de la contraction maximale observée lors de la première courbe au Sumatriptan. Les courbes sont analysées par régression non-linéaire de façon à déterminer le Eₘₐₓ (réponse maximale) et la CE₅₀ (concentration produisant 50 % de la réponse maximale). Le potentiel antagoniste des composés est estimé par calcul de la constante de dissociation K_{B} selon l'équation K_{B} = [concentration du composé en M] / (CR - 1) où CR représente le rapport des CE₅₀ du Sumatriptan en présence et en absence du composé. Le résultat est exprimé comme pA₂ = - log K_{B}. Les pA₂ des composés de l'invention sont supérieurs à 6.

Les composés de l'invention ont également fait l'objet d'un essai d'inhibition de la liaison du [³H] spiropéridol aux récepteurs sérotoninergiques 5-HT₂ du cortex cérébral de rat. Pour cet essai, on prélève les cerveaux de rats, on en dissèque le cortex et on l'homogénéise à 0°C dans 20 volumes d'un mélange contenant, par litre, 50 mmoles de tampon Tris/HCl à pH = 7.4, 120 mmoles de NaCl et 5 mmoles de KCl. On centrifuge le mélange homogène à 40000 x g pendant 10 minutes puis, à deux reprises, on récupère le culot, on le lave en le mettant en suspension dans le même mélange tampon, on l'homogénéise de nouveau et on le centrifuge. Pour terminer on dilue le culot final dans le même mélange tampon à raison de 500 mg de tissu humide pour 10 ml de tampon. On soumet alors le tissu à une incubation préalable de 10 minutes à 37 °C en présence de 10 µm/l de pargyline, puis à une incubation de 20 minutes à 37 °C en présence de ³H-spiropéridol (activité spécifique : 19 Ci par mmole) à la concentration de 0,3 nM et de composé à étudier à des concentrations allant de 0.0001 à 100 µM.
On prélève des aliquots de 1 ml que l'on filtre sous vide, on lave les filtres deux fois avec 5 ml de tampon froid, on les sèche et on mesure la radioactivité.
Pour évaluer l'activité des composés, on établit la courbe du pourcentage d'inhibition de la liaison spécifique de 3H-spiropéridol en fonction de la concentration en drogue déplaçante. On détermine graphiquement la CI₅₀, concentration qui inhibe 50 % de la liaison spécifique.
La liaison spécifique est définie comme étant la liaison déplacée par 100 µM de 5-HT.
Les CI₅₀ des composés de l'invention sont inférieurs à 1 µM.

Les résultats de ces essais ont montré que les composés de l'invention présentent des propriétés antagonistes de la sérotonine.

A ce titre, ils peuvent être utilisés dans le traitement et la prévention de diverses formes de pathologies impliquant la sérotonine, comme les hypertensions artérielle, veineuse, pulmonaire, portale, rénale ou oculaire, les ischémies cardiaque, rénale, oculaire, cérébrale, ou des membres inférieurs, l'insuffisance cardiaque, l'infarctus du myocarde, l'angor, les vasospasmes coronaires ou périphériques, les thromboses (seuls ou en adjuvants à la thrombolyse), les artérites, la claudication intermittente, les resténoses après angioplastie et différents états pathologiques associés à l'athérosclérose, aux troubles de la microcirculation ou aux dysfonctionnements pulmonaires. Ils peuvent également être utilisés, seuls ou en association avec d'autres substances dans les interventions de greffe vasculaire.

Les composés de l'invention peuvent être utilisés en association avec d'autres substances à activité cardiovasculaire ou cardiopulmonaire, telles que les antithrombotiques, les thrombolytiques, les β-bloquants, les antagonistes calciques, les antagonistes de la thromboxane, les inhibiteurs de la thromboxane synthétase.

A cet effet, ces composés peuvent être présentés sous toutes formes appropriées à l'administration orale, ou parentérale, telles que comprimés, dragées, gélules, capsules, formulations oculaires topiques en association avec des excipients convenables. Ces formes sont dosées pour permettre une administration de 0,1 mg à 1 g, une à plusieurs fois par jour.
Ils peuvent également être présentés sous toutes formes appropriées à l'administration transdermique.

## Revendications

1. Composés de formule (I) 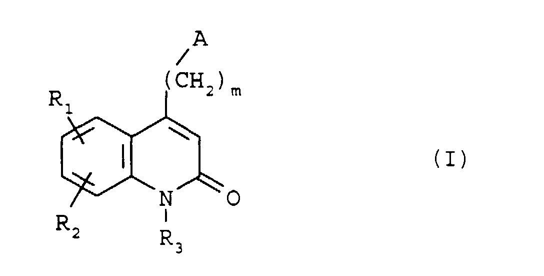 dans laquelle
A représente soit un groupe 4-(thiéno[3,2-*c*]pyridin-4-yl) pipérazin-1-yle, soit un groupe 4-(4-fluorobenzoyl)pipéridin-1-yle,
R₁ et R₂ représentent chacun indépendamment l'un de l'autre, soit un atome d'hydrogène, soit un atome d'halogène, soit un groupe amino, soit un groupe hydroxy, soit un groupe nitro, soit un groupe cyano, soit un groupe (C₁-C₆)alkyle, soit un groupe (C₁-C₆)alcoxy, soit un groupe trifluorométhyle, soit un groupe trifluorométhoxy, soit un groupe -COOH, soit un groupe -COOR₄, soit un groupe -CONH₂, soit un groupe -CONHR₄, soit un groupe -CONR₄R_{5,} soit un groupe -SR₄, soit un groupe -SO₂R₄, soit un groupe -NHCOR₄, soit un groupe -NHSO₂R₄, soit un groupe -N(R₄)₂ où R₄ et R₅ sont chacun un groupe (C₁-C₄)alkyle,
R₃ représente soit un atome d'hydrogène, soit un groupe (C₁-C₄)alkyle, soit un groupe -(CH₂)ₚOH, soit un groupe -(CH₂)ₚNH_{2,} soit un groupe -(CH₂)ₙCOOH, soit un groupe -(CH₂)ₙCOOR₄, soit un groupe -(CH₂)ₙCONH₂, soit un groupe -(CH₂)ₙCONHOH, soit un groupe -(CH₂)ₚSH, soit un groupe - (CH2)ₙSO₃H, soit un groupe -(CH₂)ₙSO₂NH₂, soit un groupe -(CH₂)ₙSO₂NHR₄, soit un groupe -(CH₂)ₙSO₂NR₄R₅, soit un groupe -(CH₂)ₙCONHR₄, soit un groupe -(CH₂)ₙCONR₄R₅, soit un groupe -(CH₂)ₚNHSO₂R₄, soit un groupe -(CH₂)ₚNHCOR_{4,} soit un groupe -(CH₂)ₚOCOR₄ où R₄ et R₅ sont chacun un groupe (C₁-C₄)alkyle,
n est égal à 1, 2, 3 ou 4, p est égal à 2, 3 ou 4 et
m est égal à 2, 3 ou 4,
ainsi que leurs sels d'addition aux acides ou aux bases pharmaceutiquement acceptables.

2. Composés selon la revendication 1 caractérisés en ce que m est égal à 2.

3. Composés selon l'une quelconque des revendications 1 et 2 caractérisés en ce que R_{1,} en position 6 ou 7 sur la quinoléinone, représente soit un atome d'hydrogène, de fluor ou de chlore, soit un groupe amino, hydroxy, nitro, cyano, (C₁-C₆)alkyle, méthoxy, trifluorométhoxy, acétylamino, méthylsulfonylamino ou diméthylamino et R₂ représente un atome d'hydrogène.

4. Composés selon l'une quelconque des revendications 1 à 3 caractérisés en ce que R₃ représente soit un atome d'hydrogène, soit un groupe (C₁-C₄)alkyle, soit un groupe -(CH₂)ₚOH, soit un groupe -(CH₂)ₙCOOH, soit un groupe -(CH₂)ₙCOOR₄, soit un groupe -(CH₂)ₙCONH₂, soit un groupe -(CH₂)ₙCONHR₄, soit un groupe -(CH₂)ₙCONR₄R₅, soit un groupe -(CH₂)ₚOCOR₄ où R₄ et R₅ sont chacun un groupe (C₁-C₄)alkyle, n est égal à 1, 2, 3 ou 4 et p est égal à 2, 3 ou 4.

5. Composés selon l'une quelconque des revendications 1 à 4 caractérisés en ce que n est égal à 1 et p est égal à 2.

6. Composés selon l'une quelconque des revendications 1 à 5, caractérisés en ce qu'ils sont choisis parmi
la 6-fluoro-1-méthyl-4-[2-(4-thiéno[3,2-*c*]pyridin-4-ylpipérazin-1-yl)éthyl]quinoléin-2(1*H*)-one,
le 7-fluoro-2-oxo-4-[2-(4-thiéno[3,2-*c*]pyridin-4-ylpipérazin-1-yl)éthyl]-1,2-dihydroquinoléine-1-acétamide,
le 7-fluoro-*N*-méthyl-2-oxo-4-[2-(4-thiéno[3,2-*c*]pyridin-4-ylpipérazin-1-yl)éthyl]-1,2-dihydroquinoléine-1-acétamide,
la 6-chloro-1-méthyl-4-[2-(4-thiéno[3,2-*c*]pyridin-4-ylpipérazin-1-yl)éthyl]quinoléin-2(1*H*)-one,
la 6-chloro-4-[2-[4-(4-fluorobenzoyl)pipéridin-1-yl]éthyl]-1-méthylquinoléin-2(1*H*)-one,
la 7-fluoro-4-[2-(4-thiéno[3,2-*c*]pyridin-4-ylpipérazin-1-yl)éthyl]quinoléin-2(1*H*)-one,
la 4-[2-[4-(4-fluorobenzoyl)pipéridin-1-yl]éthyl]-6-méthoxyquinoléin-2(1*H*)-one,
la 6-hydroxy-1-méthyl4-[2-(4-thiéno[3,2-*c*]pyridin-4-ylpipérazin-1-yl)éthyl]-quinoléin-2(1*H*)-one,
l'acétate de 2-[7-fluoro-2-oxo-4-[2-(4-thiéno[3,2-*c*]pyridin-4-ylpipérazin-1-yl)éthyl]-1,2-dihydroquinoléin-1-yl]éthyle et,
la 6-méthyl-4-[2-(4-thiéno [3,2-*c*]pyridin-4-ylpipérazin-1-yl)éthyl]quinoléin-2(1*H*)-one ainsi que leurs sels d'addition aux acides ou aux bases pharmaceutiquement acceptables.

7. Procédé de préparation des composés selon la revendication 1 caractérisé en ce que l'on fait réagir la 4-(pipérazin-1-yl)thiéno[3,2-*c*]pyridine ou la 4-(4-fluorobenzoyl)pipéridine avec un composé de formule (VII) 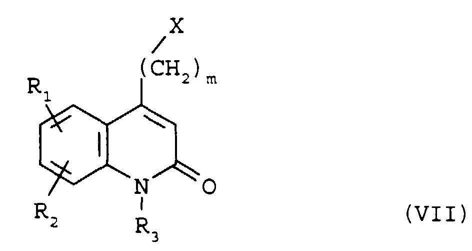 dans laquelle R_{1,} R_{2,} R₃ et m sont tels que définis dans la revendication 1 et X représente un groupe partant.

8. Procédé de préparation des composés de formule (Ib) 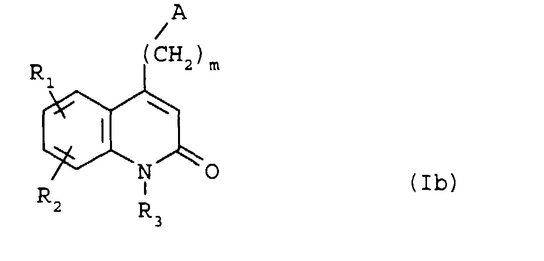 dans laquelle A, R₁, R₂ et m sont tels que définis dans la revendication 1 et R₃ est différent d'un atome d'hydrogène, caractérisé en ce que l'on fait réagir un composé de formule (Ia) 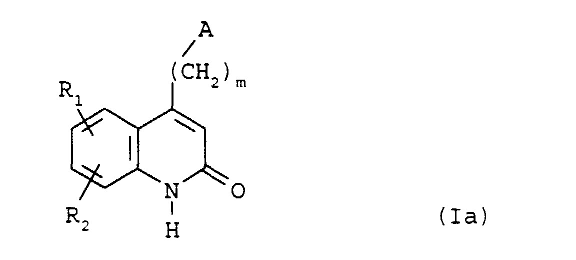 avec un agent électrophile.

9. Médicament caractérisé en ce qu'il contient un composé selon l'une quelconque des revendications 1 à 6.

10. Composition pharmaceutique caractérisée en ce qu'elle contient un composé selon l'une quelconque des revendications 1 à 6 en association avec tout excipient pharmaceutiquement acceptable.

## Patentansprüche

1. Verbindungen der Formel (I) 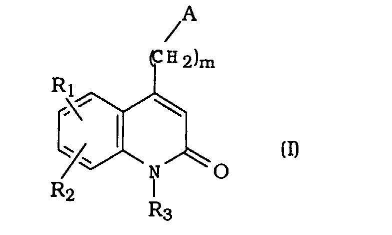 in der
A entweder eine 4-(Thieno[3,2-*c*]pyridin-4-yl)-piperazin-1-yl-gruppe oder eine 4-(4-Fluorbenzoyl)-piperidin-1-yl-gruppe,
R₁ und R₂ jeweils unabhängig voneinander entweder ein Wasserstoffatom, oder ein Halogenatom, oder eine Aminogruppe, oder eine Hydroxygruppe, oder eine Nitrogruppe, oder eine Cyanogruppe, oder eine (C₁-C₆)-Alkylgruppe, oder eine (C₁-C₆)-Alkoxygruppe, oder eine Trifluormethylgruppe, oder eine Trifluormethoxygruppe, oder eine Gruppe -COOH, oder eine Gruppe-COOR₄, oder eine Gruppe-CONH₂, oder eine Gruppe-CONHR₄, oder eine Gruppe-CONR₄R₅, oder eine Gruppe-SR₄, oder eine Gruppe -SO₂R₄, oder eine Gruppe -NHCOR₄, oder eine Gruppe -NHSO₂R₄, oder eine Gruppe -N(R₄)₂, worin R₄ und R₅ jeweils eine (C₁-C₄)-Alkylgruppe darstellen,
R₃ entweder ein Wasserstoffatom, oder eine (C₁-C₄)-Alkylgruppe, oder eine Gruppe-(CH₂)ₚOH, oder eine Gruppe -(CH₂)ₚNH₂, oder eine Gruppe -(CH₂)ₙCOOH, oder eine Gruppe -(CH₂)ₙCOOR₄, oder eine Gruppe -(CH₂)ₙCONH₂, oder eine Gruppe -(CH₂)ₙCONHOH, oder eine Gruppe -(CH₂)ₚSH, oder eine Gruppe -(CH₂)ₙSO₃H, oder eine Gruppe -(CH₂)ₙSO₂NH₂, oder eine Gruppe -(CH₂)ₙSO₂NHR₄, oder eine Gruppe -(CH₂)ₙSO₂NR₄R₅, oder eine Gruppe -(CH₂)ₙCONHR₄, oder eine Gruppe -(CH₂)ₙCONR₄R₅, oder eine Gruppe -(CH₂)ₚNHSO₂R₄, oder eine Gruppe -(CH₂)ₚNHCOR₄, oder eine Gruppe -(CH₂)ₚOCOR₄, worin R₄ und R₅ jeweils eine (C₁-C₄)-Alkylgruppe darstellen,
n 0, 1, 2, 3 oder 4, p 2, 3 oder 4 und m 2, 3 oder 4 bedeuten,
sowie deren Additionssalze mit pharmazeutisch annehmbaren Säuren oder Basen.

2. Verbindungen nach Anspruch 1, dadurch gekennzeichnet, daß m den Wert 2 besitzt.

3. Verbindungen nach irgendeinem der Ansprüche 1 und 2, dadurch gekennzeichnet, daß R₁ in der 6- oder 7-Stellung des Chinolinons entweder ein Wasserstoffatom, Fluor oder Chlor, oder eine Aminogruppe, Hydroxygruppe, Nitrogruppe, Cyanogruppe, (C₁ -C₆)-Alkylgruppe, Methoxygruppe, Trifluormethoxygruppe, Acetylaminogruppe, Methylsulfonylaminogruppe oder Dimethylaminogruppe und R₂ ein Wasserstoffatom bedeuten.

4. Verbindungen nach irgendeinem der Ansprüche 1 bis 3, dadurch gekennzeichnet, daß R₃ entweder ein Wasserstoffatom oder eine (C₁-C₄)-Alkylgruppe, oder eine Gruppe -(CH₂)ₚOH, oder eine Gruppe -(CH₂)ₙCOOH, oder eine Gruppe -(CH₂)ₙCOOR₄, oder eine Gruppe -(CH₂)ₙCONH₂, oder eine Gruppe -(CH₂)ₙCONHR₄, oder eine Gruppe -(CH₂)ₙCONR₄,R₅, oder eine Gruppe -(CH₂)ₚOCOR₄, worin R₄ und R₅ jeweils eine (C₁-C₄)-Alkylgruppe darstellen, n 1, 2, 3 oder 4 und p 2, 3 oder 4 bedeuten.

5. Verbindungen nach irgendeinem der Ansprüche 1 bis 4, dadurch gekennzeichnet, daß n den Wert 1 und p den Wert 2 besitzen.

6. Verbindungen nach irgendeinem der Ansprüche 1 bis 5, dadurch gekennzeichnet, daß sie ausgewählt sind aus 6-Fluor-1-methyl-4-[2-(4-thieno[3,2-*c*]pyridin-4-yl-piperazin-1-yl)-ethyl]-chinolin-2(1*H*)-on, 7-Fluor-2-oxo-4-[2-(4-thieno[3,2-*c*]pyridin-4-yl-piperazin-1-yl)-ethyl]-1,2-dihydrochinolin-1-acetamid, 7-Fluor-*N*-methyl-2-oxo-4-[2-(4-thieno[3,2-*c*]pyridin-4-yl-piperazin-1-yl)-ethyl]-1,2-dihydrochinolin-1-acetamid, 6-Chlor-1-methyl-4-[2-(4-thieno[3,2-*c*]pyridin-4-yl-piperazin-1-yl)-ethyl]-chinolin-2(1*H*)-on, 6-Chlor-4-[2-[4-(4-fluorbenzoyl)-piperidin-1-yl]-ethyl]-1-methylchinolin-2(1*H*)-on, 7-Fluor-4-[2-(4-thieno[3,2-*c*]pyridin-4-yl-piperazin-1-yl)-ethyl]-chinolin-2(1*H*)-on, 4-[2-[4-(4-Fluorbenzoyl)-piperidin-1-yl]-ethyl]-6-methoxychinolin-2(1*H*)-on, 6-Hydroxy-1-methyl-4-[2-(4-thieno[3,2-*c*]pyridin-4-yl-piperazin-1-yl)-ethyl]-chinolin-2(1*H*)-on, 2-[7-Fluor-2-oxo-4-[2-(4-thieno[3,2-*c*]pyridin-4-yl-piperazin-1-yl)-ethyl]-1,2-dihydrochinolin-1-yl]-ethylacetat und 6-Methyl-4-[2-(4-thieno[3,2-*c*]pyridin-4-yl-piperazin-1-yl)-ethyl]-chinolin-2(1*H*)-on sowie deren Additionssalze mit pharmazeutisch annehmbaren Säuren oder Basen.

7. Verfahren zur Herstellung der Verbindungen nach Anspruch 1, dadurch gekennzeichnet, daß man 4-(Piperazin-1-yl)-thieno[3,2-*c*]pyridin oder 4-(4-Fluorbenzoyl]-piperidin mit einer Verbindung der Formel (VII) 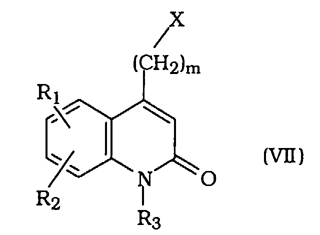 in der R₁, R₂, R₃ und m die in Anspruch 1 angegebenen Bedeutungen besitzen und X eine austretende Gruppe darstellt, umsetzt.

8. Verfahren zur Herstellung der Verbindungen der Formel (Ib) 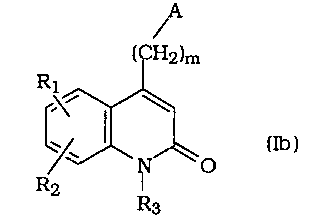 in der A, R₁, R₂ und m die in Anspruch 1 angegebenen Bedeutungen besitzen und R₃ von einem Wasserstoffatom verschieden ist, dadurch gekennzeichnet, daß man eine Verbindung der Formel (Ia) 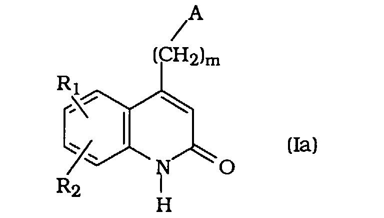 mit einem elektrophilen Mittel umsetzt.

9. Arzneimittel, dadurch gekennzeichnet, daß es eine Verbindung nach irgendeinem der Ansprüche 1 bis 6 enthält.

10. Pharmazeutische Zubereitung, dadurch gekennzeichnet, daß sie eine Verbindung nach irgendeinem der Ansprüche 1 bis 6 in Kombination mit irgendeinem pharmazeutisch annehmbaren Trägermaterial enthält.

## Claims

1. Compounds of formula (I) 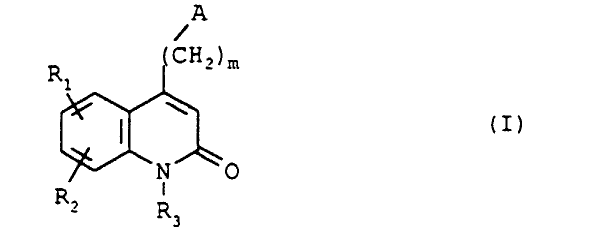 in which
A represents either a 4-(thieno[3,2-*c*]pyridin-4-yl)-1-piperazinyl group or a 4-(4-fluorobenzoyl)-1-piperidyl group,
R₁ and R₂ each represent, independently of one another, either a hydrogen atom, or a halogen atom, or an amino group, or a hydroxyl group, or a nitro group, or a cyano group, or a (C₁-C₆)alkyl group, or a (C₁-C₆)alkoxy group, or a trifluoromethyl group, or a trifluoromethoxy group, or a -COOH group, or a group -COOR₄, or a -CONH₂ group, or a group -CONHR₄, or a group -CONR₄R_{5,} or a group -SR₄, or a group -SO₂R₄, or a group -NHCOR₄, or a group -NHSO₂R₄, or a group -N(R₄)₂, where R₄ and R₅ are each a (C₁-C₄)alkyl group,
R₃ represents either a hydrogen atom, or a (C₁-C₄)alkyl group, or a group -(CH₂)ₚOH, or a group -(CH₂)ₚNH₂, or a group -(CH₂)ₙCOOH, or a group -(CH₂)ₙCOOR₄, or a group -(CH₂)ₙCONH₂, or a group -(CH₂)ₙCONHOH, or a group -(CH₂)ₚSH, or a group -(CH₂)ₙSO₃H, or a group -(CH₂)ₙSO₂NH₂, or a group -(CH₂)ₙSO₂NHR₄, or a group -(CH₂)ₙSO₂NR₄R₅, or a group -(CH₂)ₙCONHR₄, or a group -(CH₂)ₙCONR₄R₅, or a group -(CH₂)ₚNHSO₂R₄, or a group -(CH₂)ₚNHCOR₄, or a group -(CH₂)ₚOCOR₄, where R₄ and R₅ are each a (C₁-C₄)alkyl group, n is equal to 1, 2, 3 or 4, p is equal to 2, 3 or 4 and m is equal to 2, 3 or 4, as well as their addition salts with pharmaceutically acceptable acids or bases.

2. Compounds according to Claim 1, characterized in that m is equal to 2.

3. Compounds according to either of Claims 1 and 2, characterized in that R₁, at position 6 or 7 on the quinolone represents either a hydrogen, fluorine or chlorine atom, or an amino, hydroxyl, nitro, cyano, (C₁-C₆)alkyl, methoxy, trifluoromethoxy, acetylamino, methylsulphonylamino or dimethylamino group, and R₂ represents a hydrogen atom.

4. Compounds according to any one of Claims 1 to 3, characterized in that R₃ represents either a hydrogen atom, or a (C₁-C₄)alkyl group, or a group -(CH₂)ₚOH, or a group -(CH₂)ₙCOOH, or a group -(CH₂)ₙCOOR₄, or a group - (CH₂)ₙCONH₂, or a group -(CH₂)ₙCONHR₄, or a group -(CH₂)ₙCONR₄R₅, or a group -(CH₂)ₚOCOR₄ where R₄ and R₅ are each a (C₁-C₄)alkyl group, n is equal to 1, 2, 3 or 4 and p is equal to 2, 3 or 4.

5. Compounds according to any one of Claims 1 to 4, characterized in that n is equal to 1 and p is equal to 2.

6. Compounds according to any one of Claims 1 to 5, characterized in that they are chosen from
6-fluoro-1-methyl-4-[2-(4-thieno [3,2-*c*]pyridin-4-yl-1-piperazinyl)ethyl]-2(1*H*)-quinolone,
7-fluoro-2-oxo-4-[2-(4-thieno[3,2-*c*]pyridin-4-yl-1-piperazinyl)ethyl]-1,2-dihydro-1-quinolineacetamide,
7-fluoro-N-methyl-2-oxo-4-[2-(4-thieno[3,2-*c*]pyridin-4-yl-1-piperazinyl)ethyl]-1,2-dihydro-1-quinolineacetamide,
6-chloro-1-methyl-4-[2-(4-thieno [3,2-*c*]pyridin-4-yl-1-piperazinyl)ethyl]-2(1*H*)-quinolone,
6-chloro-4-[2-[4-(4-fluorobenzoyl)-1-piperidyl]ethyl]-1-methyl-2(1*H*)-quinolone,
7-fluoro-4-[2-(4-thieno [3,2-*c*]pyridin-4-yl-1-piperazinyl)ethyl]-2(1*H*)-quinolone,
4-[2-[4-(4-fluorobenzoyl)-1-piperidyl]ethyl]-6-methoxy-2(1*H*)-quinolone,
6-hydroxy-1-methyl-4-[2-(4-thieno [3,2-*c*]pyridin-4-yl-1-piperazinyl)ethyl]-2(1*H*)-quinolone,
2-[7-fluoro-2-oxo-4-[2-(4-thieno [3,2-*c*]pyridin-4-yl-1-piperazinyl)ethyl]-1,2-dihydro-1-quinolyl]ethyl acetate and,
6-methyl-4-[2-(4-thieno [3,2-*c*]pyridin-4-yl-1-piperazinyl)ethyl]-2(1*H*)-quinolone, as well as their addition salts with pharmaceutically acceptable acids or bases.

7. Process for preparing the compounds according to Claim 1, characterized in that 4-(1-piperazinyl)thieno[3,2-*c*]pyridine or 4-(4-fluorobenzoyl)piperidine is reacted with a compound of formula (VII) 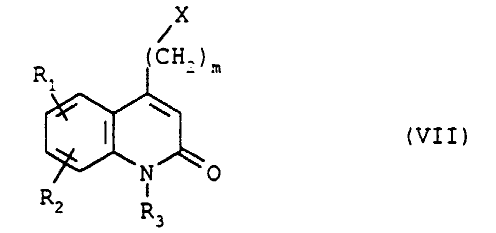 in which R₁, R₂, R₃ and m are as defined in Claim 1 and X represents a leaving group.

8. Process for preparing the compounds of formula (Ib) 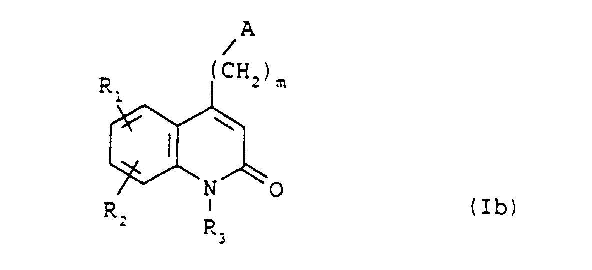 in which A, R₁, R₂ and m are as defined in Claim 1 and R₃ is other than a hydrogen atom, characterized in that a compound of formula (Ia) 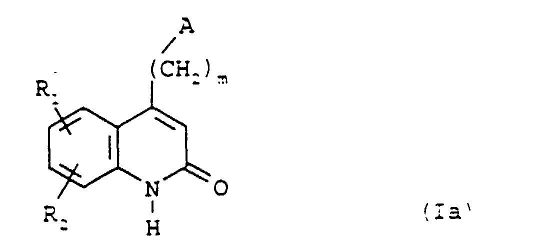 is reacted with an electrophilic agent.

9. Medicinal product, characterized in that it contains a compound according to any one of Claims 1 to 6.

10. Pharmaceutical composition, characterized in that it contains a compound according to any one of Claims 1 to 6, in combination with any pharmaceutically acceptable excipient.
